# EUROPEAN PATENT SPECIFICATION

(11) **EP 3 914 383 B1**
(45) Date of publication and mention of the grant of the patent: **15.07.2026**
(21) Application number: 20701071.1
(22) Date of filing: 24.01.2020
(51) Int. Cl.: B01J 2/22, B30B 11/18

(54) **METHOD OF DRY GRANULATION USING A ROLLER COMPACTOR**
VERFAHREN ZUR TROCKENGRANULATION MIT HILFE EINER WALZENPRESSE
PROCÉDÉ DE GRANULATION SECHE À L'AIDE D'UN COMPACTEUR À ROULEAUX

(30) Priority: 24.01.2019 EP 19153436
(43) Date of publication of application: 01.12.2021
(73) Proprietor: Novo Nordisk A/S, 2880 Bagsværd (DK)
(72) Inventor: VILHELMSEN, Thomas, 2880 Bagsværd (DK); SØRENSEN, Martin, Nobert, 2880 Bagsværd (DK)
(86) International application number: PCT/EP2020/051696
(87) International publication number: WO 2020/152304

(56) References cited:
- CN-A- 105 056 832
- CN-A- 105 056 832
- CN-U- 204 865 754
- JP-A- S6 293 099
- US-A1- 2011 220 745
- US-A1- 2015 072 926
- US-A1- 2015 072 926
- US-A1- 2017 173 911

## Description

The present invention relates to a method of producing a solid pharmaceutical composition comprising a) producing granules comprising a salt of N-(8-(2-hydroxybenzoyl)amino)caprylic acid, and a continuous method of producing ribbons comprising a salt of N-(8-(2-hydroxybenzoyl)amino)caprylic acid.

### BACKGROUND OF THE INVENTION

Granules for the forming of solid dosage forms, such as tablets, capsules or sachets can be produced by dry granulation using a roller compactor. Granules are used as an intermediate in the manufacturing of various dosage forms, e.g. tablets, capsules, and sachets. The granules may be used as is or in combination with other excipients.

Roller compactors are used for dry granulation of powders. Dry granulation can be accomplished by several means. When a roller compactor is applied for dry granulation, the process is often referred to as roller compaction. The roller compactor compacts dry powder into ribbons by applying mechanical force on a moving bed of the dry powder using a set of rollers. The resulting ribbons are milled to form granules. Roller compaction is therefore a continuous process transforming dry powder into granules via a ribbon intermediate.

WO2008/056021 A2 relates to roller compaction of powders for producing granulate for tabletting which may comprise an API. The document describes that although dry granulation would in many cases appear to be the best way to produce products such as tablets containing APIs, it has been relatively little used because of the challenges in producing the desired kind of granules as well as managing the granulated material in the manufacturing process. The dry granulation methods known in the prior art produce granules that are seldom usable in a tablet manufacturing process. Conflicting process design parameters often lead to compromises where some qualities of the resulting granule product may be good, but other desirable qualities are lacking or absent. For example, the flow characteristics of the granules may be insufficient, the non-homogeneity of the granules may cause segregation in the manufacturing process or capping in tablets, or some of the granules may exhibit excessive hardness, all of which can make the tableting process very difficult, slow and sometimes impossible. Furthermore, the bulk granules may be difficult to compress into tablets.

US2011/0220745 and US6623756 also describes roller compactors for dry granulating powders for forming a granulate mass for tableting.

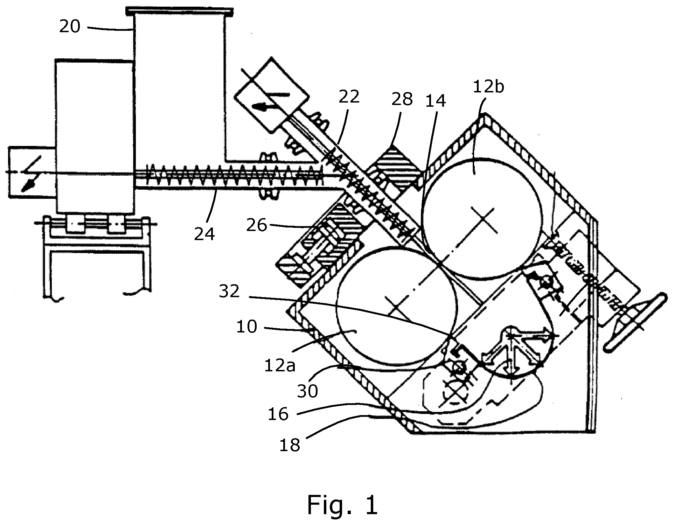

US5509612 discloses a roller compacter for continuous mechanical shaping of a particulate material. The roller compactor is shown in figure 1 and comprises a housing 10, a pair of pressing rolls 12 forming a pressing space 14 and enclosed in the housing 10. The pair of pressing rolls 12 consists of two pressing rollers 12a, 12b, wherein each of the pressing rollers has a rotation axis. The compactor further comprises a rotor 16 arranged in the housing below the pair of pressing rolls 12, and a sieve insert 18 mounted in the housing 10 adjacent to the rotor 16. Powder or a mixture of different powders is conveyed from a proportioning device 20 into the stuffing screw 22 during operation of a proportioning screw 24, which can be regulated. The stuffing screw 22 takes over the powder and conveys it into the pressing space 14 of the pressing rollers 12a, 12b, which run against one another. A hydraulic cylinder 26 is fastened to the stationary housing 10, acted upon by pressure, and draws the pressing rollers 12a and 12b toward one another via a tension stirrup 10. The powder is aerated between the rollers 12 and compressed to form a scab. A stripper 30, with an edge 32, can be provided to avoid double pressing of products, by removing products which may possibly adhere to the rollers 12a, 12b.

CN 105056832A discloses a roller compacter comprising a housing consisting of plates (18 and 19) and baffles (21 and 22) installed at the discharge end of the feed mechanism (16) sealing of the space towards the two rollers.

US 2015/072926 discloses granules and/or a pharmaceutical composition comprising the sodium salt of N-(8-(2-hydroxybenzoyl)amino)caprylic acid (SNAC) and the use of a roller compactor.

A continuous flow of powder, ribbons, and granules through the roller compactor is therefore crucial in order to avoid build-up and ultimately blockage of the roller compactor resulting in unacceptable manufacturing conditions. Build-up within the roller compactor may result as a consequence of e.g. poor flowability, cohesive forces, and electrostatic charging of the powder, ribbons, and granules. Build-up within the roller compactor has a negative impact on the technical performance of the compaction process, product quality, and process yield.

Some of these above mentioned problems can be addressed by adding lubricants to the powder. Lubricants tend to be hydrophobic, so their levels need to be optimized. Underlubricated blends tend to flow poorly and show sticking problems, and over-lubricated blends can adversely affect tablet hardness, dissolution rate, as well as tablet friability.

CA 2938916 describes a method to prevent sticking/caking, and at the same time reduce the amount of internal lubricant to avoid the negative effects of too much internal lubricant. In CA 2938916 this problem is addressed by a method of external lubrication of the press roll of a roller compaction device by continuous coating of the rotating pressing rollers, wherein the pressing rollers are coated with a thin layer of a lubricant in a continuous and solvent-free manner.

### SUMMARY OF THE INVENTION

However, despite the above mentioned advances, it is an object of the present invention to provide further methods for enabling continuous dry granulation in a roller compactor. It is a further object of the invention to secure consistent quality of granules, and to be able to provide granules of a consistent quality obtained by continuous processing. It is a further object of the invention to provide methods enabling long term continuous processing without interruptions due to cleaning or maintenance, and whereby the devices and the methods provide cost effective production conditions.

The inventors have solved the problem of build-up of material/product by improving the dry granulation equipment and thereby enable continuous production of granules of a sufficiently high quality and homogeneity for an extended time period. As described herein, build-up of granules can be prevented by including an agitator preventing build-up of material below the granulator. Alternatively, or additionally a roller rim stripper can be included to prevent build-up or accumulation of material on the roller rim of the pressing rollers, and thereby prevent that the pressing rollers slow down.

Disclosed is a roller compactor for production of granules by dry granulation, wherein the roller compactor comprises: an inlet and an outlet, a pair of pressing rollers comprising a first and a second pressing roller, and wherein the first pressing roller comprises roller rims, wherein the roller compactor further comprises a granulator, a sieve, and exit slides below the sieve leading towards the outlet, wherein the roller compactor further comprises: a) roller rim strippers (250) and/or b) an agitator (270) adapted for agitating material tending to accumulate at the slides (215.2).

Hereby is disclosed a roller compactor adapted for preventing build-up or accumulation in the roller compactor. The agitator prevents build-up of powder, granules and ribbons below the granulator, and upstream to the granulator and below the pressing rollers. The roller rim strippers prevent build-up on the roller rims, and thereby prevents a slowing down of the roller rims, and consequently a different pressing time of the inflowing powder. In combination the agitator below the granulator ensures that material can be led away from the pressing rollers, whereby also the tendency to accumulate on the roller rims decreases, and the roller rim strippers ensure a continuous strip off of the product, i.e., the agitator and the roller rim strippers can work individually and in concert to prevent build-up of ribbons and granules in the roller compactor and thereby prevent a negative impact on product quality.

According to the invention, there is provided a method of producing ribbons or granules comprising a salt of N-(8-(2-hydroxybenzoyl)amino)caprylic acid, wherein the method comprises:
a) providing a powder comprising a salt of N-(8-(2-hydroxybenzoyl)amino)caprylic acid;
b) compacting the powder into ribbons by using a roller compactor wherein the roller compactor comprises: an inlet (201) and an outlet (202), a pair of pressing rollers (212) comprising a first (212a) and a second pressing roller (212b), wherein the first pressing roller (212a) comprises roller rims (213) and wherein the roller compactor further comprises roller rim strippers (250).

In a further embodiment the method is for continuous production of granules by dry granulation.

In a further embodiment the method is for production of granules by continuous dry granulation.

In a further embodiment adhered material is removed at the edge of the rim stripper, the stripping edge 255, and guided to the outlet

In a further embodiment the stripping edge is less than 180 degrees away from the pressing space on the roller rim circumference in the direction of the rotation.

In a further embodiment the roller compactor further comprises a roller scraper with an edge. Such roller scraper or roller scrapers are adapted to avoid double pressing of the material by scraping of material at one or both of the pressing rollers.

In a further embodiment the roller scraper edge and the stripping edge are both within 90 degrees on the circumference of the pressing roller/roller rim.

In a further embodiment the roller scraper and the rim strippers are integrated in a single unit. In such embodiments the roller scraper edge and the stripping edge are preferably within 45 degrees on the circumference of the pressing roller/roller rim.

In a further embodiment the roller scraper and the rim strippers are separate units.

In a further embodiment the roller scraper edge and the stripping edge (255) are less than 90 degrees away from the pressing space on the circumference of the pressing roll- er/roller rim in the direction of rotation.

In a further embodiment the roller rim strippers (250) extend over more than 45 degrees of the circumferential of the roller rims 213.

In a further embodiment the roller rim strippers are adapted for stripping off material tending to accumulate or build-up on the roller rims, and thereby to lead the stripped off material towards the outlet.

In a further embodiment the agitator is adapted for agitating material tending to accumulate below the sieve insert.

In a further embodiment the roller compactor comprises a channel insert unit adapted to interface the roller rims and provide a clearance between the channel insert unit and the roller rims.

In a further embodiment, the agitator is an air blowing insert agitating the product and utilizing the air blow and gravity to lead the agitated product towards the outlet.

In another embodiment is provided a method of producing granules comprising a salt of N-(8-(2-hydroxybenzoyl)amino)caprylic acid comprising the following steps:
- (i) providing a material comprising a salt of N-(8-(2-hydroxybenzoyl)amino)caprylic acid;
- (ii) dry granulating the material by using a roller compactor according to the above described roller compactor;
- (iii) obtaining granules comprising a salt of N-(8-(2-hydroxybenzoyl)amino)caprylic acid.

Hereby is provided a method of producing a granule comprising a salt of N-(8-(2-hydroxybenzoyl)amino)caprylic acid by continuously preventing accumulation of material in the roller compactor, wherein the accumulation is prevented by agitation and strip off during dry granulation.

In a further embodiment the roller rim strippers are adapted for stripping off material tending to accumulate or build-up on the roller rims, and thereby lead the stripped off material towards the outlet.

In a further embodiment, the agitator is adapted for agitating material tending to accumulate below the sieve insert.

In further embodiments, the methods herein are continuous for more than 5 minutes, such as for more than 10 minutes, such as for more than 20 minutes, such as for more than 30 minutes, such as for more than 45 minutes, such as for more than 1 hour, such as for more than 2 hours, such as for more than 3 hours, such as for more than 5 hours, such as for more than 8 hours.

In another embodiment is provided a method wherein the roller compactor comprises: a flow channel for conveying a material from an inlet to an outlet through a pressing space, wherein the material comprises the ingredients and/or the excipients, wherein the material can be in different physical forms comprising the form of powder, particles and the form of granules;
- wherein the roller compactor further comprises: a housing, a pair of rollers for forming the pressing space enclosed by the housing, wherein the pair of pressing rollers comprises two pressing rollers, and wherein the first pressing roller comprises roller rims;
- wherein the roller compactor further comprises: a granulator arranged in the housing downstream to the pair of pressing rollers, and a sieve insert mounted in the housing adjacent and downstream to the granulator;
- wherein the roller compactor further comprises an agitator adapted for agitating material tending to accumulate at the exit slides below the sieve insert, and thereby lead the agitated material towards the outlet.

In a further embodiment the roller compactor is for production of granules by continuous dry granulation.

In a further embodiment the roller compactor is for continuous production of granules by dry granulation.

In a further embodiment the agitator is an air blowing insert agitating the material and utilizing the air blow and gravity to lead the agitated material towards the outlet.

In a further embodiment, the agitator is a vibrating plate agitating the granules and utilizing vibrational energy and gravity to lead the agitated material towards the outlet.

In a further embodiment, the agitator is an automatically movable mechanical brush, scraper or swiping arm agitating the granules and utilizing the automatic movement and gravity to lead the agitated material towards the outlet.

In a further embodiment, the agitator is adapted to counteract an accumulation of material at the exit slide below the sieve and the granulator, and thereby counteract the upstream accumulation at the slides below the pressing rollers.

In a further embodiment, the material is characterized by poor flowability due to cohesive forces, or electrostatic charging.

In a further embodiment, the poor flowability can be observed as a tendency to accumulate at the exit slides below the granulator and the sieve insert and below the pair of rollers.

In a further embodiment, the poor flowability is defined as a production stop, in a reference roller compactor without the agitator, due to a material accumulation at the exit slides below the granulator and sieve insert before the completion of a production cycle without cleaning the slides below the granulator or without agitation of the granules below the granulator of a period longer than a time within the interval 1 minute to 120 minutes, or preferably longer than 6 minutes.

In a further embodiment, for the purpose of defining poor flowability, the reference roller compactor is defined as a roller compactor wherein the mechanical parts, with the exception of the agitator, are identical to the mechanical parts of the embodiment according to the present disclosure.

In a further embodiment the roller compactor is adapted for ensuring consistent quality of the granules by counteracting accumulation of material in the roller compactor.

In a further embodiment the roller compactor is adapted for ensuring consistent quality of the granules by extending the period of a continuous production cycle, without interruption by cleaning cycles.

In a further embodiment the roller compactor is adapted for enhancing the yield of the dry granulation process by counteracting accumulation of material in the roller compactor.

In a further embodiment the roller compactor is adapted for enhancing the dry granulation process by extending the period of a continuous production cycle, without interruption by cleaning cycles.

In a further embodiment the roller compactor is adapted for dry granulation of a powder comprising a salt of N-(8-(2-hydroxybenzoyl)amino)caprylic acid.

In a further embodiment the roller compactor is adapted for production of granules comprising a salt of N-(8-(2-hydroxybenzoyl)amino)caprylic acid.

In a further embodiment the roller compactor is adapted for forming the powder into ribbons, and for forming the ribbons into granules.

In another embodiment is provided a method of producing granules comprising a salt of N-(8-(2-hydroxybenzoyl)amino)caprylic acid according to the present disclosure, the method comprises:
- (i) providing a powder comprising a salt of N-(8-(2-hydroxybenzoyl)amino)caprylic acid;
- (ii) dry granulating the powder by using a roller compactor according to the roller compactor described above;
- (iii) continuously during dry granulation, agitating and guiding agitated material towards the outlet (202).

In another embodiment is provided a method for production of ribbons by compaction of ingredients and/or excipients, wherein the roller compactor comprises: a flow channel for conveying a material from an inlet to an outlet through a pressing space, wherein the material comprises the ingredients and/or the excipients, wherein the material can be in different physical forms comprising the form of powder, particles and the form of granules;
- wherein the roller compactor further comprises: a housing, a pair of rollers for forming the pressing space enclosed by the housing, and wherein the pair of pressing rollers comprises a first and a second pressing roller, and wherein the first pressing roller comprises roller rims;
- wherein the roller compactor further comprises roller rim strippers, wherein the roller rim strippers are adapted for stripping off material tending to accumulate on the roller rims, and thereby lead the stripped off material towards the outlet.

In a further embodiment the roller compactor is for production of ribbons by continuous compaction.

In a further embodiment the roller compactor is for continuous production of ribbons by compaction.

In a further embodiment, the roller compactor comprises a channel insert unit adapted to interface the roller rims and provide a clearance between the channel insert unit and the roller rims.

In a further embodiment, the roller compactor further comprises: a granulator arranged in the housing downstream to the pair of pressing rollers, and a sieve insert mounted in the housing adjacent and downstream to the granulator.

In a further embodiment, the channel insert unit comprises side walls adapted to be inserted upstream and adjacent to the pair of rollers and for interfacing with the first pressing roller, wherein the channel insert unit is adapted to provide a channel portion from the inlet to the pressing space between the pair of rollers, and wherein the side walls are adapted to interface the roller rims and provide clearance between the channel unit insert and the roller rims.

In a further embodiment, the channel insert unit further comprises a first lower curved surface adapted to interface the roller rims and provide the clearance.

In a further embodiment, the clearance is adapted to minimize friction upon rotation of the roller rims but also to restrict powder from escaping the intended flow path through the pressing space during the dry granulation.

In a further embodiment, the roller rims and the channel insert unit are adapted to create friction, in response to formation of a material layer due to material accumulation on the roller rims, wherein the material layer exceeds a threshold, whereby rotational speed of the rollers will slow down.

In a further embodiment, the roller rim strippers are adapted to ensure that the material layer is prevented in exceeding the threshold.

In a further embodiment, the roller rims comprise two roller rims and the roller rim strippers comprise two roller rim strippers.

In a further embodiment, the material is characterized by poor flowability due to cohesive forces, or electrostatic charging.

In a further embodiment, the poor flowability can be observed as a tendency to accumulate on the roller rim stripper.

In a further embodiment, the poor flowability can be defined as a production stop, in a reference roller compactor without the roller rim strippers, due to a material accumulate on the roller rims before the completion of a production cycle without cleaning or stripping of the roller rims of a period longer than a time within the interval 1 minute to 120 minutes, or preferably longer than 30 minutes.

In a further embodiment, for the purpose of defining poor flowability, the reference roller compactor is defined as a roller compactor wherein essentially all the mechanical parts, with the exception of the roller rim strippers, are identical to the embodiment according the present disclosure.

In a further embodiment, the roller compactor is adapted for ensuring consistent quality of the granules by counteracting accumulation of material in the roller compactor, and thereby ensure that the rotational speed of the first roller is not reduced.

In a further embodiment, the roller compactor is adapted for enhancing the yield of the dry granulation process by counteracting accumulation of material in the roller compactor.

In a further embodiment, the roller compactor is adapted for enhancing the yield of the dry granulation process by extending the period of a continuous production cycle, without interruption by cleaning cycles.

In a further embodiment, the roller compactor is adapted for dry granulation of a powder comprising a salt of N-(8-(2-hydroxybenzoyl)amino)caprylic acid.

In a further embodiment, the roller compactor is adapted for production of ribbons comprising a salt of N-(8-(2-hydroxybenzoyl)amino)caprylic acid.

In a further embodiment, the roller compactor is adapted for forming the powder into ribbons, and wherein the ingredients and/or excipients further composes the ribbons.

In another embodiment is provided a method of producing ribbons comprising a salt of N-(8-(2-hydroxybenzoyl)amino)caprylic acid according to the present disclosure, the method comprises:
- (i) providing a powder comprising a salt of N-(8-(2-hydroxybenzoyl)amino)caprylic acid;
- (ii) dry granulating the powder by using a roller compactor according to the roller compactor described above;
- (iii) continuously during dry granulation, agitating and guiding agitated material towards the outlet.

In another embodiment is provided, a method for production of granules by dry granulation of ingredients and/or excipients, wherein the roller compactor comprises: a flow channel for conveying a material from an inlet to an outlet through a pressing space, wherein the material comprises the ingredients and/or the excipients, wherein the material can be in different physical forms comprising the form of powder, particles and the form of granules;
- wherein the roller compactor further comprises: a housing, a pair of rollers for forming the pressing space enclosed by the housing, and wherein the pair of pressing rollers comprises two pressing rollers, and wherein the first pressing roller comprises roller rims;
- wherein the roller compactor further comprises: a granulator arranged in the housing downstream to the pair of pressing rollers, and a sieve insert mounted in the housing adjacent and downstream to the granulator;
- wherein the roller compactor further comprises an agitator adapted for agitating material tending to accumulate on the slides below the sieve insert, and thereby lead the agitated product toward the outlet, and roller rim strippers, wherein the roller rim strippers are adapted for stripping off material tending to accumulate on the roller rims, and
thereby lead the stripped off material towards the outlet.

In another embodiment is provided a method of producing granules comprising a salt of N-(8-(2-hydroxybenzoyl)amino)caprylic acid according to the present disclosure, the method comprises:
- (i) providing a powder comprising a salt of N-(8-(2-hydroxybenzoyl)amino)caprylic acid;
- (ii) dry granulating the powder by using a roller compactor 200 according to the roller compactor described above;
- (iii) continuously during dry granulation, agitating and guiding agitated material towards the outlet 202.

In further embodiments the roller compactors and methods described herein are for continuous production of granules comprising a salt of N-(8-(2-hydroxybenzoyl)amino)caprylic acid.

In another embodiment is provided, a method of producing a solid pharmaceutical composition comprising the steps of
a) producing granules comprising a salt of N-(8-(2-hydroxybenzoyl)amino)caprylic acid according to the above described method
b) preparing said solid pharmaceutical formulation using the granules obtained in a).

In one embodiment the method comprise a step of blending said salt of N-(8-(2-hydroxybenzoyl)amino)caprylic acid and a lubricant, such as magnesium stearate prior to step a).

In a further embodiment the method comprise a step of blending said granules obtained in a) with a lubricant, such as magnesium stearate prior to step b).

In a further embodiment the method of producing a solid pharmaceutical composition comprising the steps of:
a) blending a salt of N-(8-(2-hydroxybenzoyl)amino)caprylic acid with a lubricant, such as magnesium stearate
b) producing granules comprising said blend of salt of N-(8-(2-hydroxybenzoyl)amino)caprylic acid and magnesium stearate according to the above described method
c) blending said granules obtained in b) with a lubricant, such as magnesium stearate
d) preparing said solid pharmaceutical formulation using the blend obtained in c).

In one embodiment the granules comprise 1-5 mg magnesium stearate per 100 mg SNAC.

In a further embodiment the solid pharmaceutical formulation comprises a pharmaceutical active ingredient.

In a further embodiment the method of producing a solid pharmaceutical composition comprising the steps of:
a) blending a salt of N-(8-(2-hydroxybenzoyl)amino)caprylic acid, a pharmaceutical active ingredient and a lubricant, such as magnesium stearate
b) producing granules comprising said blend of salt of N-(8-(2-hydroxybenzoyl)amino)caprylic acid, a pharmaceutical active ingredient and magnesium stearate according to the above described method
c) preparing said solid pharmaceutical formulation using the blend obtained in b).

In a further embodiment the method of producing a solid pharmaceutical composition comprising the steps of:
a) blending a salt of N-(8-(2-hydroxybenzoyl)amino)caprylic acid with a lubricant, such as magnesium stearate
b) producing granules comprising said blend of salt of N-(8-(2-hydroxybenzoyl)amino)caprylic acid and magnesium stearate according to the above described method
c) blending said granules obtained in b) with a composition comprising a pharmaceutical active ingredient and
d) preparing said solid pharmaceutical formulation using the blend obtained in c).

In a further embodiment the method of producing a solid pharmaceutical composition comprising the steps of:
a) blending a salt of N-(8-(2-hydroxybenzoyl)amino)caprylic acid with a lubricant, such as magnesium stearate
b) producing a first type of granules comprising said blend of salt of N-(8-(2-hydroxybenzoyl)amino)caprylic acid and magnesium stearate according to the above described method
c) producing a second type of granules comprising a pharmaceutical active ingredient,
d) blending said granules obtained in b) with said granules obtained in c) and
e) preparing said solid pharmaceutical formulation using the blend obtained in d).

In a further embodiment the method of producing a solid pharmaceutical composition comprising the steps of:
a) blending a salt of N-(8-(2-hydroxybenzoyl)amino)caprylic acid with a lubricant, such as magnesium stearate
b) producing a first type of granules comprising said blend of salt of N-(8-(2-hydroxybenzoyl)amino)caprylic acid and magnesium stearate according to the above described method
c) producing a second type of granules comprising a pharmaceutical active ingredient,
d) blending said granules obtained in b) with said granules obtained in c) and preparing said solid pharmaceutical formulation using the blend obtained in d).

In further such embodiments the pharmaceutical active ingredient is semaglutide

### BRIEF DESCRIPTION OF THE DRAWINGS

In the following embodiments of the invention will be described with reference to the drawings:
Figure 1 shows a roller compactor of prior art.
Figure 2A shows a cross section of a roller compactor according to the present disclosure.
Figure 2B shows the roller compactor of figure 2A in perspective view.
Figure 2C shows details of the pressing rollers and the roller scrapers of the roller compactor of figure 2A.
Figure 2D and 2E show details of the channel insert unit of the roller compactor of figure 2A.
Figure 2F and 2G show details of roller rims and roller rim strippers of the roller compactor of figure 2A.
Figure 2H and 2I show details of the air blowing insert, the granulator and the sieve of the roller compactor of figure 2A.
Figure 2J show pictures of accumulating powder in the roller compactor of figure 2A without an agitator. The pictures are taken after production and before cleaning. Accumulation of powder at the components illustrates the problem preventing continuous production.
Figure 3A, 3B and 3C illustrate alternative embodiments of roller rim strippers, agitators and sieve inserts according to the present disclosure.

In the figures like structures are mainly identified by like reference numerals. Numbers e.g. 200, 201, 202 are used to denote features on the drawing. Numbers combined with letters e.g. 212a, 212b are used to denote features with a similar function, and these features can be referred to individually as 212a and 212b or in common as 212. Further details of a feature can be denoted by a number followed by a dot and a running number of 1 or 2 digits. Further details of a feature can be denoted by a number combined with a letter followed by a dot and a running number of 1 or 2 digits.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member is used for a given component it can be used to define a unitary component or a portion of a component, having one or more functions.

In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

It will also be understood that, although the terms first, second, etc. may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

As used herein, the term "continuous" is used to describe a period without interruption or uninterrupted extension. A "continuous method" is used to describe a method which can be continued without interruption due to external conditions determined by the nature of the process, e.g., the process is stopped due to required cleaning of the equipment or the process is stopped due to excessive accumulation which blocks the process or prevents the functioning of the equipment.

A "pharmaceutical active ingredient" is as used herein preferably a GLP-1 receptor agonist, such as a GLP-1 analogue comprising a fatty acid or fatty diacid substituent extending the half-life of the peptide. Example compounds are described in WO2006/097537, WO2011/080103 and WO2012/140117 and includes semaglutide (WO2006/097537, Example 4), Example 2 of WO2011/080103 or Example 31 of WO2012/140117. In a preferred embodiment said pharmaceutical active ingredient is semaglutide. In a further preferred embodiment said pharmaceutical active ingredient is N^{ε27}-[2-[2-[2-[[2-[2-[2-[[(4S)-4-carboxy-4-[10-(4-carboxyphenoxy)decanoylamino]-butanoyl]amino] ethoxy]ethoxy]acetyl]amino]ethoxy]ethoxy]-acetyl], N^{ε36}-[2-[2-[2-[[2-[2-[2-[[(4S)-4-carboxy-4-[10-(4-carboxyphenoxy)decanoylamino]-butanoyl]amino]-ethoxy]-ethoxy]acetyl]amino]ethoxy]ethoxy]acetyl]-[Aib8,Glu22,Arg26,Lys27, Glu30,Arg34,Lys36]-GLP-1-(7-37)-peptidyl-Glu-Gly. In a further preferred embodiment said pharmaceutical active ingredient is named N^{ε26}{2-[2-(2-{2-[2-(2-{(S)-4-Carboxy-4-[10-(4-carboxyphenoxy)decanoylamino]butyrylamino}-ethoxy)ethoxy]acetylamino}ethoxy)ethoxy]acetyl}, N^{ε37}-{2-[2-(2-{2-[2-(2-{(S)-4-carboxy-4-[10-(4-carboxyphenoxy)decanoylamino]butyrylamino}ethoxy)-ethoxy]acetylamino}ethoxy)ethoxy]-acetyl}-[Aib8,Arg34,Lys37]GLP-1(7-37)-peptide.

### Granules comprising a salt of N-(8-(2-hydroxybenzoyl)amino)caprylic acid

In the present disclosure, granules comprising a salt of N-(8-(2-hydroxybenzoyl)amino)caprylic acid are used as an intermediate for the manufacturing of an orally administered dosage, wherein the dosage can be in the form of a tablet, a capsule, or a sachet. The granules may be used as is or in combination with other excipients.

The granules comprise a salt of N-(8-(2-hydroxybenzoyl)amino)caprylic acid, such as a potassium, calcium or sodium salt of N-(8-(2-hydroxybenzoyl)amino)caprylic acid. In a preferred embodiment the salt is sodium N-(8-(2-hydroxybenzoyl)amino)caprylic acid (SNAC). In one embodiment the granules comprises a salt of N-(8-(2-hydroxybenzoyl)amino)caprylic acid, such as salt of N-(8-(2-hydroxybenzoyl)amino)caprylic acid and a lubricant, such as magnesium stearate. In one embodiment the granules comprise SNAC and magnesium stearate. In one embodiment the granules comprise 1-5 mg magnesium stearate per 100 mg SNAC.

SNAC is a delivery agent and has shown to improve the bioavailability of semaglutide as described in WO2012/080471. WO 2013/139694 further describes a preferred dosage form where the solid composition is made up of two types of granulate, a first type comprising SNAC and a second type comprising semaglutide.

In alternative embodiments the dosage form may comprise a single type of granules comprising a salt of N-(8-(2-hydroxybenzoyl)amino)caprylic acid (such as SNAC) and a pharmaceutical active ingredient, and wherein said single type of granules are produced according to the present invention.

In some embodiments the granules comprising salt of N-(8-(2-hydroxybenzoyl)amino)caprylic acid further comprises a lubricant, such as magnesium stearate. In some embodiments the granules comprising salt of N-(8-(2-hydroxybenzoyl)amino)caprylic acid further comprises a filler, such as microcrystalline cellulose. Accordingly, the granules produced according to the invention comprising a salt of N-(8-(2-hydroxybenzoyl)amino)caprylic may further comprise a lubricant and optionally a filler.

In one embodiment the invention relates to granules produced according to the methods described herein, and further to any solid pharmaceutical dosage form comprising granules produced according to the invention. Such solid pharmaceutical dosage form additionally comprises a pharmaceutical active ingredient. In a preferred embodiment said pharmaceutical active ingredient is semaglutide.

In some embodiments the term "granule" refers to powder or particles gathered into larger particles. Such granules can be used to produce a pharmaceutical composition by blending the granules with further excipient. Such further excipients may be in the form of powder, particles or in the form of further granules, such as a second granule. Finally, further excipients may be added by blending prior to finalizing the pharmaceutical composition. Pharmaceutical compositions produced according to the invention may thus comprise a granular part and an extra-granular part. The pharmaceutical active ingredient may be part of either the granular part or the extra-granular part.

Some embodiments of the present disclosure relates to a solid pharmaceutical composition comprising granules of a first type and optionally a second type, wherein said first type of granules comprises a salt of N-(8-(2-hydroxybenzoyl)amino)caprylic acid and said second type of granules comprises a pharmaceutical active ingredient, and wherein said first type of granules are produced according to the present invention. In some embodiments the granules comprising salt of N-(8-(2-hydroxybenzoyl)amino)caprylic acid further comprises a lubricant, such as magnesium stearate. In some embodiments the granules comprising salt of N-(8-(2-hydroxybenzoyl)amino)caprylic acid further comprises a filler, such as microcrystalline cellulose. Accordingly, the granules may further comprise a lubricant and optionally a filler.

In embodiments comprising a second type of granules comprising the pharmaceutical active ingredient, the second type of granules may further comprise a filler, such as microcrystalline cellulose. In some embodiments the second type of granules further comprises a binder, such as povidone. Accordingly, the second type of granules may further comprise a filler and optionally a binder. In some embodiments the second type of granules further comprises an extragranular lubricant, such as magnesium stearate.

In some embodiments the first type of granules comprising a salt of N-(8-(2-hydroxybenzoyl)amino)caprylic acid does not contain a pharmaceutical active ingredient. In some embodiments the second type of granules comprising a pharmaceutical active ingredient does not contain a salt of N-(8-(2-hydroxybenzoyl)amino)caprylic acid.

In some embodiments the first type of granules comprises a salt of N-(8-(2-hydroxybenzoyl)amino)caprylic acid and no pharmaceutical active ingredient, and the second type of granules comprises a pharmaceutical active ingredient and no salt of N-(8-(2-hydroxybenzoyl)amino)caprylic acid.

In some embodiments of the present disclosures relates to a solid pharmaceutical composition comprising granules comprising a salt of N-(8-(2-hydroxybenzoyl)amino)caprylic acid and optionally a second type of granules wherein said first type of granules are produced according to the present invention. The solid pharmaceutical composition further comprises a pharmaceutical active ingredient either, and said active ingredient may as described above be comprised by the granules comprising a salt of N-(8-(2-hydroxybenzoyl)amino)caprylic acid or alternatively included in the pharmaceutical composition in separate granules or extra-granulate.

In the present disclosure, a roller compactor generally used for dry granulation of a powder, and it is particularly used for dry granulation of powder comprising a salt of N-(8-(2-hydroxybenzoyl)amino)caprylic acid. The roller compactor compress the dry powder into ribbons by applying mechanical force on a moving bed of the dry powder using a set of rollers. The resulting ribbons are milled to form granules. The roller compaction is therefore a continuous process transforming the dry powder into granules via a ribbon intermediate.

### Roller compaction

The present disclosure relates to a process of roller compaction, wherein a material is conveyed from an inlet to an outlet. The material comprises the active ingredients and/or excipients of the manufacturing process. The material has different forms depending on the processes that have been applied, but the material has substantially the same composition of ingredients and/or excipients through the process. The transformation from one form to another is accomplished by a processing step. The different forms of the material in the disclosed method of roller compaction comprises: powder, ribbons and granules. However, the material may comprise other forms or intermediate forms. At all stages and in all forms from the inlet to outlet the material can also be referred to as a product to indicate the value or purpose of the material. For the disclosed roller compaction process the powder is the starting product, which has been provided as a blend of powders, if there is more than one ingredient and/or excipient. The ribbons are defined as an intermediate product obtained by compression of the blend of powders, having the appearance of a long narrow strip. The granules are defined as the end product or final product and is obtained by comminution of the ribbons. However, for a further process step of e.g. tabletting the granules, the granules can be regarded as a starting product or an intermediate product, and the tablet will be regarded as the final product.

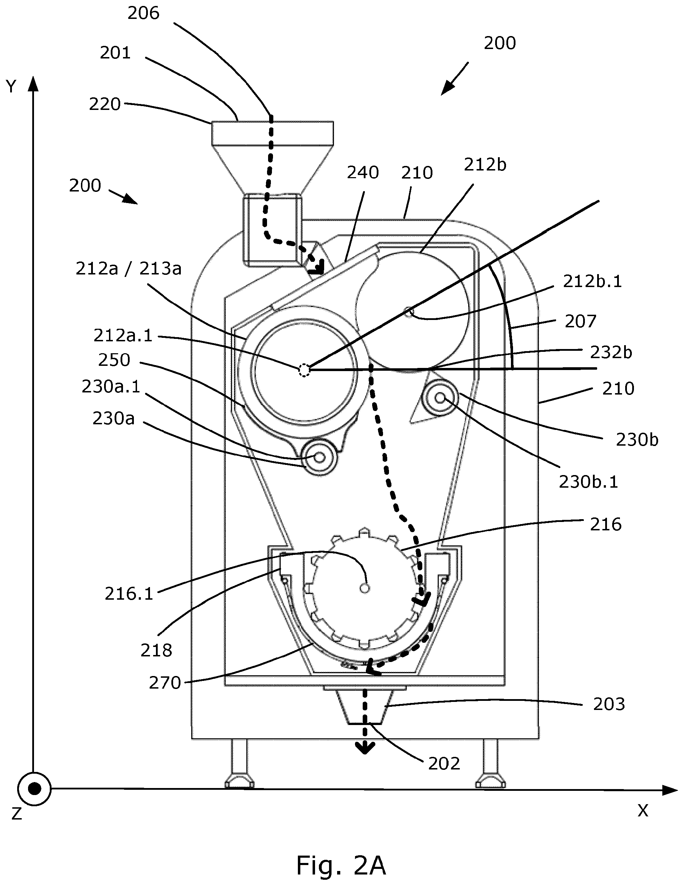

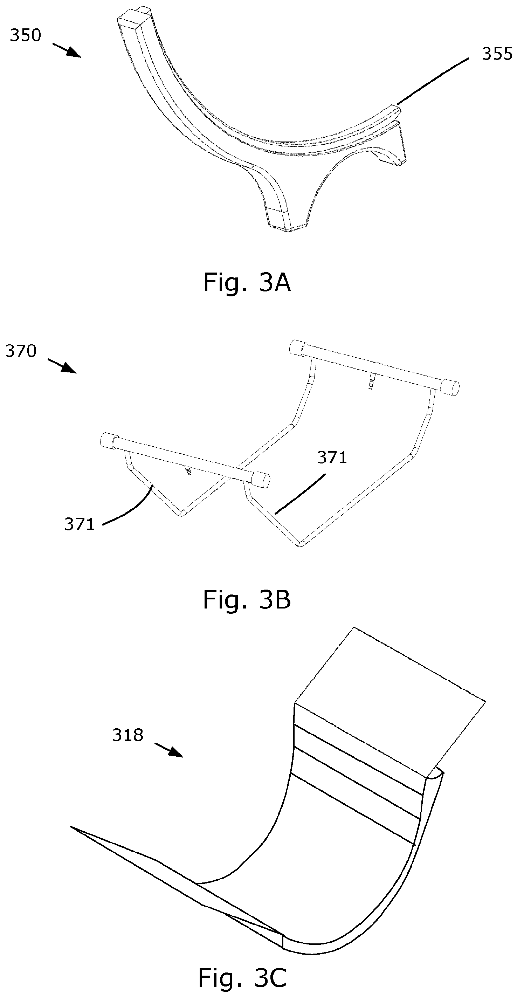

Figures 2A to 2J illustrate a roller compactor 200 and equipment 250 and 270 inserted. The illustrated equipment 250 and 270 are a roller rim stripper 250 and an air blowing insert 270. In the present disclosure, the roller compactor wherein at least the air blowing insert has been inserted is referred to as a modified roller compactor. Figure 2A illustrates the so-called MINI-PACTOR^{®} from Gerteis with the equipment 250 and 270 inserted. The MINI-PACTOR is particularly suitable for small scale production and laboratory development. Figures 3A and 3B illustrate in detail the equipment to modify a MACRO-PACTOR^{®}, which is a roller compactor also from Gerteis, into a modified roller compactor according to the present disclosure. The MACRO-PACTOR is designed for development, pilot projects and up to full-scale production. Figure 3B illustrates an air blowing insert 370, or more generic an agitator, which is adapted to fit under a sieve insert 318 for the MACRO-PACTOR and clear the exit slides below the sieve. The sieve insert is illustrated in figure 3C.

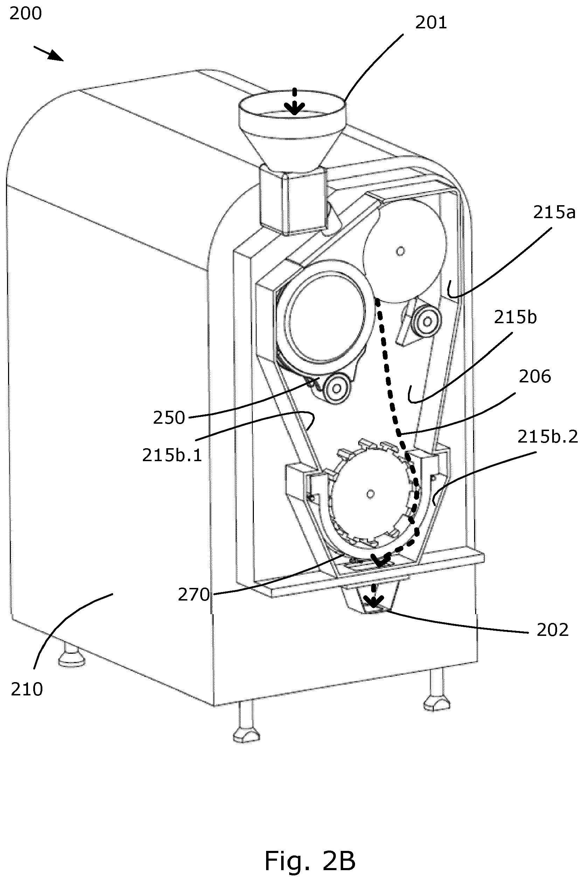

Figure 2A illustrates a cross section and figure 2B illustrates a perspective view of the roller compactor 200 comprising a flow channel for conveying the product from an inlet 201 to an outlet 202, wherein the product can be in the form of powder, ribbons or granules. The powder is provided as a blend of excipients before compaction. The ribbons and the granules are formed by the powder, and the composition does therefore depend on the composition of the blend. The dashed arrow 206 illustrates a flow path through the flow channel. The arrow 206 is divided into three pieces, wherein the discontinuation between the pieces illustrates that the flow path is covered from this point of view by an illustrated structure. A three dimensional Cartesian coordinate system, with three axes denoted by the letters X, Y and Z are also shown on figure 2A, to provide a frame of reference to describe directions and relative positions of the technical features. The axes are generally aligned with generally plane portions of the outer surfaces of a housing 210, wherein the X-axis defines the width, the Y-axis defines the height, and the Z-axis defines the depth. The plane portions are typically main portions defining more than 50 % of the outer surface. A lateral direction is used to describe a direction in the XY-plane, for a given Z coordinate, and a transverse direction is used to describe a direction in the Z-direction and normal to the XY-plane. The relative positions below and above are used to indicate relative positions on the X-axis. For a given Y-coordinate, a direction from a first position referred to as below towards a second position referred to as above is defined by the direction of the X-axis.

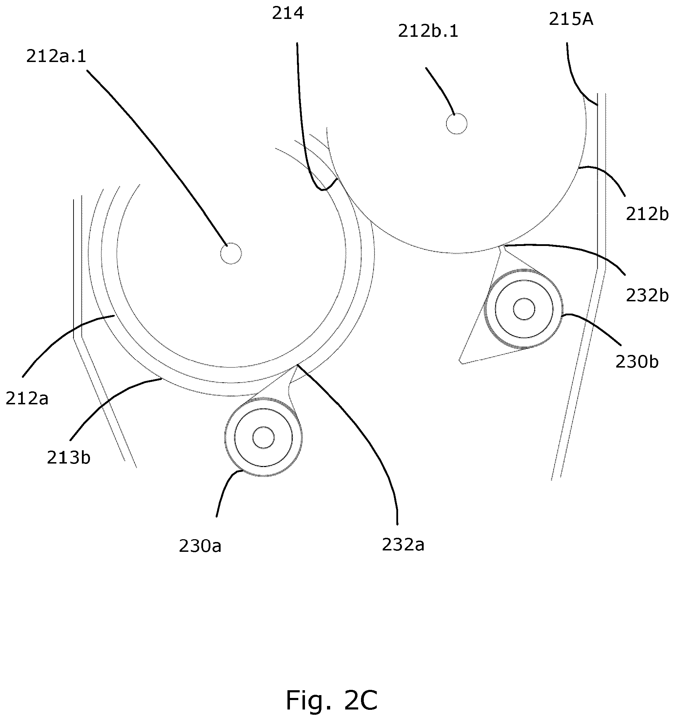

The roller compactor 200 illustrated in figures 2A and 2B comprises the housing 210, a powder feeding unit 220, a pair of rollers 212 forming a pressing space 214 (illustrated in figure 2C). As the product passes the pressing space, a portion of the product is transformed from powder to ribbons. The pressing space is enclosed in the housing 210. The pair of pressing rollers 212 comprises two pressing rollers 212a, 212b, wherein each of the pressing rollers is mounted on rotatable shafts 212a.1, 212b.1 having a rotational axis in the Z-direction. The position of the shaft 212a.1 is indicated with a dotted circle, as the shaft is hidden from the point of view in the illustration. The roller compactor 200 further comprises a granulator 216 arranged in the housing 210 below the pair of pressing rollers 212, and a sieve insert 218 mounted in the housing 210 adjacent to the granulator 216. As the product passes the granulator, a portion of the product is transformed from ribbons to granules. The magnitude of the transformed portion depends on the process parameters of the roller compactor. The granulator 216 is mounted on a rotatable shaft 216.1 adapted to rotate in the Z-direction. In the illustrated example, the rotational axes of the pressing rollers and the granulator 216 are parallel or normal to the plane portions of the outer surface of the housing 210, therefore, the flow channel can also be described with a width in the X-direction, a height in the Y-direction and a depth in the Z-direction. The housing 210 comprises wall structures 215 defining an internal volume and provide the outer boundaries for the product being processed by the pressing rolls and the granulator. Figure 2B illustrate a wall structure portion 215a providing the side walls and a wall structure portion 215b providing the rear wall. A front wall structure is to be mounted on the side walls to provide the front wall. The front wall is not illustrated, but is typically provided in a transparent material or with windows to enable of inspection of the process.

As also illustrated in figures 2A and 2B the internal volume confined by the wall structure 215 is in fluid communication with an exit chute 203. In the illustrated example a ledge is formed in the connection between the internal volume and the exit chute, however, ledges and surfaces prone to accumulation of material is to be avoided in the design of the flow path. In other words wall structure and connections should be streamlined.

A blend or mixture of powder can be conveyed from a proportioning device at the inlet 201 during operation of a stuffing screw and a proportioning screw (screws and proportioning device not shown on the figure). The powder is conveyed from the inlet and into the pressing space 214 of the pressing rollers 212a, 212b, which run against each other. The pressing roller 212a is mounted on a laterally fixed first rotatable shaft and the roller 212b is mounted on a laterally movable second rotatable shaft, which is laterally biased towards the first shaft, whereby a biasing force provides a constant compression on the pressing space 214. The first rotatable shaft 212a.1 is mounted below and laterally spaced from the second rotatable shaft 212b.1, whereby a direction through the first and second axle defines an angle 207 to the X-axis. In this way the roller 212a is adapted to draw a larger fraction of product into the pressing space 214 than the roller 212b.

Roller scrapers 230a, 230b with edges 232a, 232b are provided to avoid double pressing of the product, by removing excess of product adhering to the rollers 212a, 212b (the edge 232a is not shown on figures 2A and 2B). The roller scrapers 230a, 230b are mounted on shafts 230a.1, 230b.1, wherein the shafts are having a longitudinal extension in the Z-direction.

Figure 2C illustrate the roller scrapers 230a and 230b in more detail, where the edge 232a can be clearly viewed as well as a roller rim 213b mounted on the rear side of the roller 212a. Another roller rim 213a is to be mounted on the front side of the roller 212a, but only the roller rim 213b is shown on figure 2C. The roller rims 213 provide side walls for the pressing space 214. The roller rims 213 together with the rollers 212restrict the conveyed or conducted product from escaping the intended flow path 206 from the inlet, through the pressing space and towards the outlet 202.

### Channel insert unit

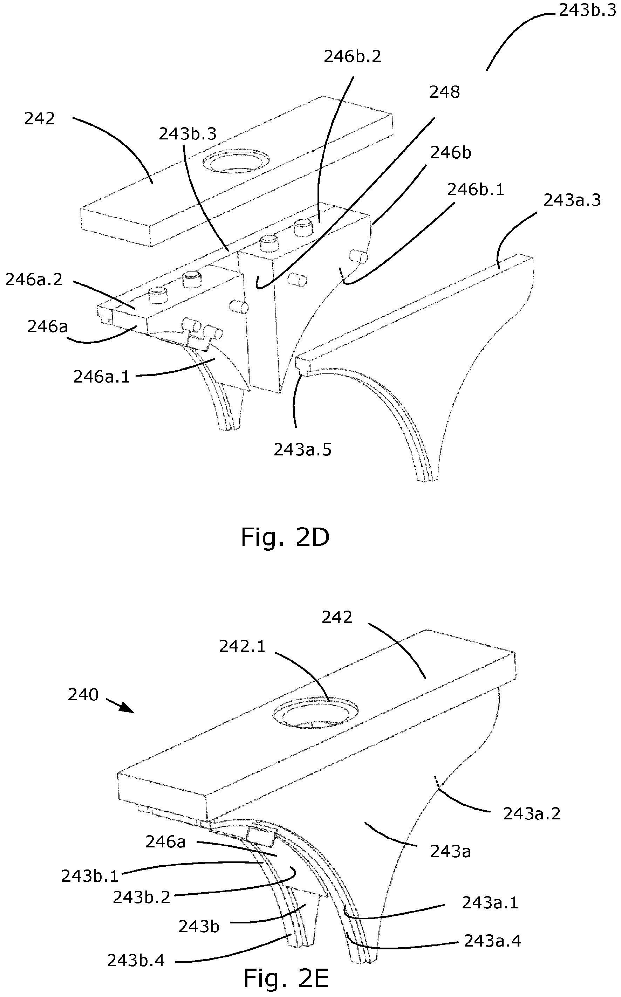

Figures 2D and 2E illustrate a channel insert unit 240 adapted to be inserted adjacent to the pair of rollers 212, i.e., the channel insert is adapted to interface with the pair of rollers 212 and roller rims 213. The channel insert unit 240 is adapted to provide a channel portion 248 from a feeding unit 220 to the pressing space 214. The channel insert unit comprises a top plate 242 with a through hole 242.1 in fluid communication with the channel portion 248. The top plate 242 is adapted to be rigidly mounted to the housing 210. The channel insert unit 240 further comprises channel center walls 246a, 246b and channel side walls 243a, 243b. The channel center walls 246 and the channel side walls 243 define the channel portion 248 with an outlet in fluid communication with the pressing space 214, and an inlet in fluid communication with the feeding unit 220. Each of the channel center walls 246 comprises a curved lower surface 246a.1, 246b.1 adapted to interface either the roller 212a or the roller 212b. Each of the channel center walls 246 further comprises an upper flat surface 246a.2, 246b.2 adapted to interface a lower flat surface of the top plate 242. The channel center walls 246 are adapted to be rigidly mounted to the top plate 242 to prevent powder from escaping the intended flow path 206. The side walls are adapted to be flexibly connected to the top plate. The flexibility can be embodied by inserting a flexible element between the top plate and the side walls 243 (not shown on the figure) or the flexible element can be an integral part of the side wall. By implementing a flexible element the side plates will be biased towards a resting position adjacent to the pressing rollers, when a force is applied on the side walls towards the top plate. In order to ensure a unilateral movement of the sidewalls 243 relative to the center walls 246 and top plate 242, unilateral guiding means can be provided between the center walls and the side walls, e.g., a pin can ride in an elongated aperture.

Each of the channel side walls 243a, 243b comprises a first lower curved surface 243a.1, 243b.1 adapted to interface either the front roller rim 213a or the rear roller rim 213b. Each of the channel side walls 243a, 243b further comprises a second lower curved surface 243a.2 exposed to the internal volume downstream to the pressing space 214 of the roller compactor 200, and an upper surface 243a.3 (see figure 2D) adapted to sealingly interface the lower surface of the top plate 242.

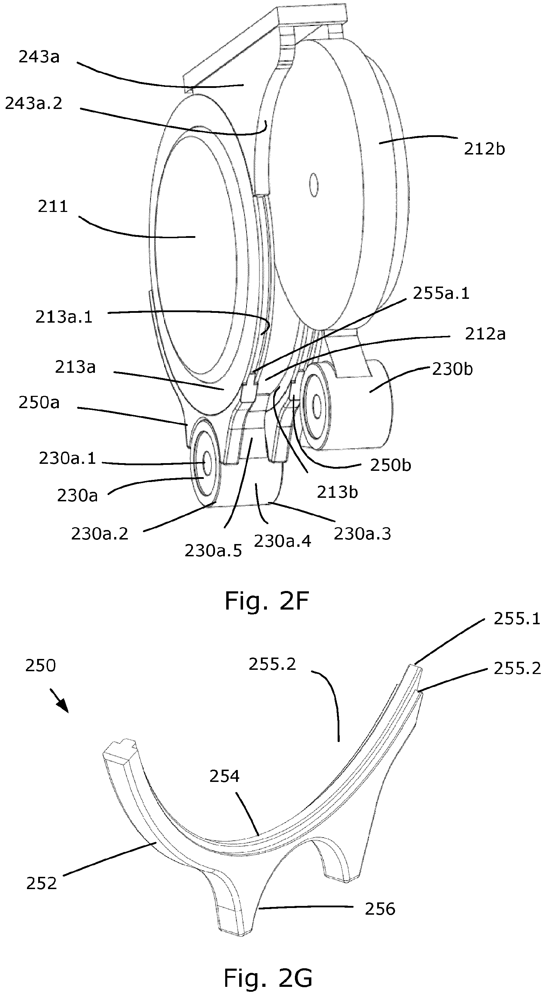

Each of the first lower curved surfaces 243a.1, 243b.1 of the channel side walls 243 further comprises a first curved guide structure 243a.4, 243b.4 adapted to cooperate with a curved circumferentially extending second curved guide structure 213a.1, 213b.1 on the roller rims 213 (see figure 2F), whereby the roller rims 213 can be guided by the side walls 213 during rotation. However, a clearance is provided between the side wall and the roller rims, in order to avoid contact and friction relating to contact between the two parts, in response to rotation and relative movement. The first curved guide structure 243.4 can be a protruding circumferential portion, and the second curved guide structure 213.1 can be a circumferential track or vice versa. The first lower curved surface 243.1 extends from a rounded edge 243a.5. When the side walls 243 is mounted adjacent to the roller rims 213, the lower curved surface 243.5 is positioned at the top of the roller rim. During roller compaction the edge 243.5 will to some extend block excess material accumulated on the roller rims. However, due to the flexible mounting of the side wall and due to the position of the edge relative to the roller rims 213, material released from the rim will not be removed to a sufficient degree, as gravity acts towards the upper surface of the roller rim. The flexible mounting ensures that the roller can still rotate, when a product layer starts to build up on the rim, and the rounded surface tends to compact the accumulated layer rather than stripping it off.

### Roller rim stripper

Figure 2F shows the front roller rim 213a, the rear roller rim 213b, the pressing rollers 212a, 212b, the front roller rim stripper 250a, the rear roller rim stripper 250b, and the front side wall 243a with the second lower surface 243a.2 exposed to the internal volume downstream to the pressing space 214. The front side walls are mounted to the top plate and interfaces the roller rims. The figure illustrates the front side wall 243a interfacing the front roller rim 213a, whereby the first lower surface 243a.1 is covered by the front roller rim 213a. The figure illustrates the second curved guide structure 213a.1 of the front roller rim 213a, which is adapted to cooperate with the first curved guide structure 243a.4. The roller rim strippers 250 are mounted to strip the roller rims for excess product adhered to the radial surface or the roller rims, which is discussed in further detail later in the application. The adhered product is removed at the stripping edge 255 (see below) where the roller rim meets the rim strippers in the rotation from the pressing space 214. The roller rim strippers 250 may extend over more than 45 degrees of the circumferential of the roller rims 213. Figure 2F also shows a covering plate 211 covering bolts used to assemble the pressing roller 212a with the roller rims 213. A small clearance is provided between the covering plate 211 and the wall structure portion 215 providing the front wall.

Figure 2G illustrates further detail of the roller rim stripper 250. The roller rim strippers 250 comprises a curved main body 252 having a curvature corresponding to the curvature of the roller rims 213, and thereby adapted to follow the radial surface of the roller rims 213. The roller rim strippers 250 further comprises a third curved guide structure 254 with a curvature corresponding to the second curved guide structure 213.1. The third curved guide structure is thereby adapted to follow the second curved guide structure 213.3 of the roller rims 213. In the illustrated embodiment, the third curved guide structure is a protruding guide structure and is thereby adapted to follow the second curved guide structure, when it is formed as a track. However, in some embodiments the third curved guide structure could be a track and the second curved guide structure a protruding guide structure. The third curved guide structure 254 comprises a stripping edge 255.1, and the main body comprises a stripping edge 255.2. The combined stripping edge 255 is adapted to strip off the product tending to accumulate on the roller rims 213. In some embodiments, the combined stripping edge is formed as a blocking element, wherein a front surface comprising the stripping edge is normal to a tangent of the radial surface of the roller rim, wherein the tangent is defined at the position closest to the stripping edge, when mounted in the roller compactor. In some embodiments and as illustrated on the figures, the front surface comprising the stripping edge forms an acute angel to a tangent of the roller rim, wherein the tangent is defined as above. The front surface with the acute angel lifts the material of rather than just blocking the material. The stripping edge 255 is positioned at a lower portion of the roller rim, whereby stripped material will be forced away from the radial surface of the roller rim. The stripping edge of the rim stripper is positioned after the pressing space in the direction of rotation. To further guide the stripped off material to the outlet the stripping edge (255) is placed before the lowermost of the roller rim and the stripping edge may be located before and above the bottom of the roller rim. In the compactor comprising roller scrapers (230) and roller rim strippers (250) the roller scraper edge 232a and rim stripper edge (255) are in close proximity. For material adhered on the lower portion gravity points away from the radial surface, and for material adhered on the upper portion gravity points towards the radial surface. In this context upper and lower portion of the roller rim is defined relative to the gravitational field, which in the illustrated example of figure 2A is opposite to the X-axis. The main body 252 together with the third guide structure 254 following the circumferential extension of the main body 252, provides a physical barrier against deposition and accumulation on the radial surface of a lower portion of the roller rim 213. The lower portion of the roller rim will, during roller compaction, be more exposed to product tending to accumulate as this portion is closer to the illustrated flow path 206. In effect, the lower portion of the roller rim is less exposed and less prone for adhering to dispersed product. The roller rim stripper further comprises a fourth curved guide structure 256 with a curvature corresponding to the stripper shaft 230a.1 or to a skirt portion 230a.2 of the stripper 230a. The fourth curved guide structure is adapted to guide the roller rim stripper into the correct position and to be supported by the stripper shaft 230a.1 or the skirt portion 230a.2 of the stripper 230. The roller rim strippers 231 are fixed, when the roller compactor 200 is assembled and ready for roller compaction. The roller rim strippers are adapted to be clamped in the Z-direction by the housing 210. Due to the clamping and the snugly fitting between the third and the second guide structure, and between the fourth supported guide structure and the stripper shaft 230a.1 or the skirt portion 230a.2 of the stripper 230, the roller rim strippers 213 stay in place during roller compaction.

The roller rim strippers 250 for the roller compactor counteracts the build-up of powder and ribbons on the roller rims 213 as the roller rim strippers 250 ensures that powder and ribbons continuously are prevented from entering the roller rims and removed from the roller rims. The entry of powder and ribbons on the roller rims is prevented by the roller rim strippers physically blocking the access by extending along the circumference of the roller rim 213. The removal is caused by the stripper design in the form for a stripping edge, i.e., an acute angle. The stripping edge is formed on the upstream edge of the roller rim stripper 250. A small clearance is provided between the roller rim and the roller rim stripper to prevent damage of the roller rim stripper.

Referring back to figure 2F, the figure illustrates the scraper 230a mounted on the shaft 230a.1. In the illustrated embodiment the scraper 230a comprises a center portion 230a.4 with a protruding portion 230a.5 extending towards the surface of the pressing roll 212a. However, a small gap is provided between the protruding portion and the surface in order not to damage the pressing roller or the scraper. The scraper 230a further comprises a skirt portion 230a.2 and a skirt portion 230a.3 on each side of the center portion 230a.4. As described above the roller rim strippers 250 can be supported by these skirt portions. However, in some embodiments the scraper 230a only comprises the center portion 230a.4. For such an embodiment, the roller rim strippers 250 are mounted on each side of the center portion 230a.4, and the roller rim strippers are supported directly by the shaft 230a.1.

### Air blowing insert

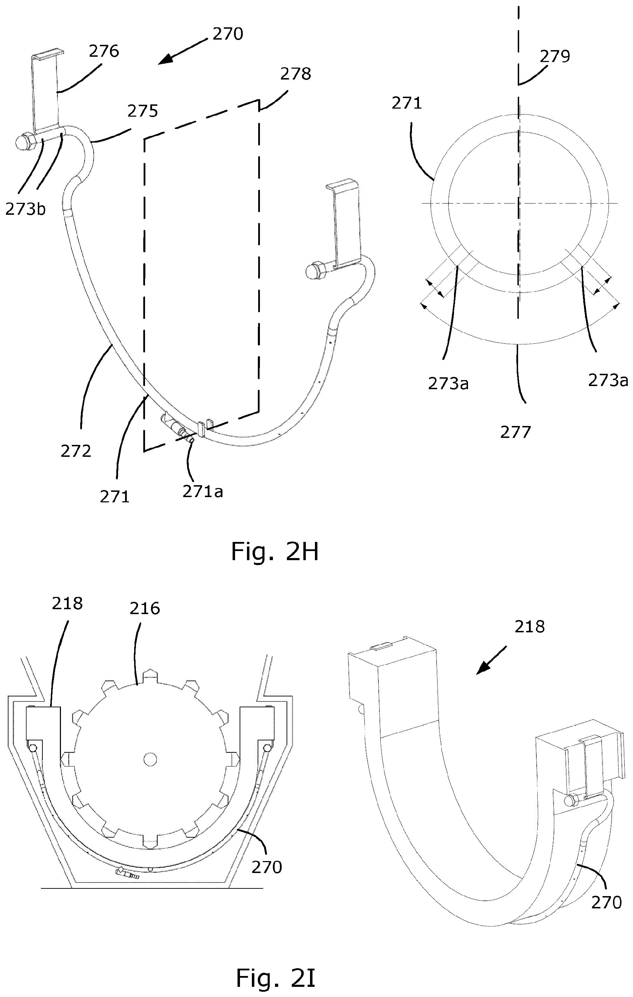

Figure 2H left panel illustrates the air blowing insert 270 for the roller compactor 200 illustrated in figure 2A and 2B. The air blowing insert 270 comprises a main body 272 comprising a curved tube 271 with a curvature corresponding to a curvature of the sieve 218. The air blowing insert comprises nozzles 273. Figure 2H right panel illustrates a cross section of the air blowing insert, wherein the cross section illustrates the orientation of the nozzles 273. The cross section is provided for the tubular portion 271. Even though the air blowing insert is referred to as an air blowing insert, it is envisioned that other gasses or mixture of gasses can be utilized in the current invention. In preferred embodiment the gas or mixture of gasses is inert and does not react with the product.

Figure 2I left panel illustrates the sieve 218 inserted below the granulator 216 in the roller compactor 200. The curvature of the sieve 218 corresponds to the curvature of the granulator 216 (i.e., the curvature of an imaginary circle circumscribing the granulator 216) and the sieve 218 is thereby adapted to follow the circumference of the granulator 216 and to be inserted below the granulator. The curvature of the air blowing insert 270 is seen to follow the curvature of the sieve insert. Figure 2I right panel further illustrates a perspective view of the air blowing insert 270 inserted below the sieve insert 218. As the curvature of the air blowing insert 270 corresponds to the curvature of the sieve insert, the air blowing unit is adapted to follow the circumference of the sieve.

Again with reference to figure 2H, the figure illustrates that in some embodiments the nozzles 273 can be positioned symmetrically around a symmetry plane 278. The symmetry plane 278 is parallel to the ZY-plane for the air blowing insert inserted into the roller compactor of figure 2A. In some embodiments, the nozzles are positioned equidistantly from the sieve and are thereby adapted to provide a symmetric air flow from the sieve towards the exit chute 203, whereby the air blowing insert is adapted induce a symmetric air flow to agitate the granules and enhance the flow of granules towards the exit chute. The effect of the generated airflow is therefore to prevent accumulation and build-up of granules below the granulator.

With further reference to figure 2H right panel, in some embodiments the nozzles are positioned symmetrically around the symmetry line 279 and are pointing downwards in order to induce a flow in the direction of the gravitational field and the exit chute. As illustrated in the right panel of figure 2H, the symmetry line and the nozzles defined an angle 277, which in some embodiments is 45-100 degrees, and in a preferred embodiment 90 degrees. In some embodiments the diameter of the nozzles is 0.1 to 3 mm, and in preferred embodiments the diameter is 1 mm. The tube 271 also comprises an air inlet 271a, and when air (or other mixture of gasses) under pressure flows into the inlet, the air continues to flow along the longitudinal extension of the tube and out of the outlet nozzles 273, whereby the air enters the space between the sieve 218 and the wall structure 215. The air blowing insert 270 for the roller compactor prevents build-up of ribbons and granules in the granulator below the sieve and beneath the rollers 212 as an air flow from the air blowing insert 270 forces granules underneath the granulator in the direction of the exit chute 203 of the roller compactor. This ensures that the granules exit the roller compactor at a rate higher or equivalent to the granulation rate and thus preventing that granules block the passage beneath the granulator. This in turn prevents that ribbons and granules build up in the granulator 216 and beneath the rollers 212. If product starts to accumulate below the granulator, the problem of accumulation will propagate upstream in the roller compactor.

With further reference to figure 2H, in some embodiments the air blowing insert 270 comprises a bend portion 275 with nozzles 273b. The nozzles 273 provided on the tube portion 271 are more specifically denoted 273a. For the air blowing insert inserted into the roller compactor illustrated in figure 2A, the bend portion 275 is bending and extending in the Z-direction and thereby enables the nozzles 273b to be positioned further apart around a symmetry plane parallel to the XY-symmetry plane and is thereby adapted to induce a flow along the depth of the flow channel. The air blowing insert 270 further comprises positioning parts 276 adapted to interface and engage with the roller compactor 200. In the illustrated example the positioning parts 276 is a bracket adapted to engage the sieve 218, which is rigidly mounted to the roller compactor, whereby the air blowing insert is fixedly positioned relative to the sieve 218, the flow channel and the exit chute.

A continuous flow of powder, ribbons, and granules through the roller compactor is crucial in order to avoid build-up and ultimately blockage of the roller compactor resulting in unacceptable manufacturing. Ideally, the continuous flow of product is provided in a steady state condition wherein the mass inflow balances the mass outflow. In reality the balance will fluctuate around this ideal condition, but over a long production cycle with continuous flow the fraction between the total mass inflow and the total mass outflow will converge. Build-up of product within the roller compactor may result as a consequence of e.g. poor flowability, cohesive forces, and electrostatic charging of the powder, ribbons, and granules. Build-up of product within the roller compactor has a negative impact on the process technical performance of the roller compactor, product quality, and process yield. Especially build-up of product in the roller compactor of powder and ribbons on rollers and their rims and build-up of ribbons and granules beneath rollers and granulators have a high negative impact on product quality.

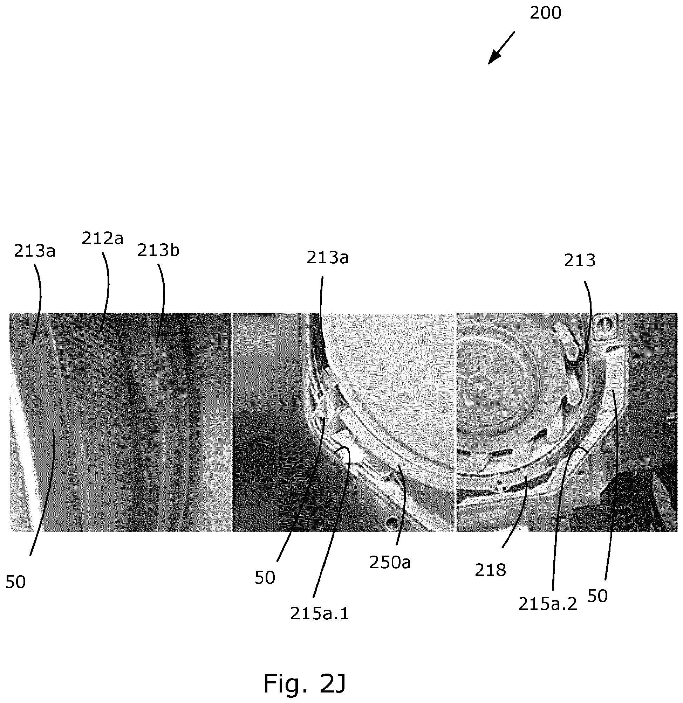

Figure 2J shows pictures of parts of particular interest, when considering the problem of product build-up within the internal volume of the roller compactor 200. The pictures are obtained after roller compaction with a modified roller compactor comprising a roller rim stripper but no air blowing insert. The left panel shows the roller 212a, and the roller rims 213a and 213b with product 50. The shown amount of adhered product 50 is insufficient to block or slow the roller rim and thereby a production stop is avoided. The left panel shows a picture after a production, wherein the roller scraper 230 and the roller rim stripper 250 have stripped product from the pressing roller and the roller rims during roller compaction. The middle panel shows a portion of the pressing roller 212a and the roller rim stripper 250a. The middle panel further shows the problem of accumulated product 50 below the roller 212a. The accumulation is due to the omission of an air blowing insert during roller compaction. The right panel shows accumulation of product 50 below the granulator 216 and the sieve insert 218 also due to the omission of the air blowing insert in the roller compactor. When product 50 accumulates below the sieve 218, the problem of accumulation propagates upstream in the roller compactor and forces a production stop.

By providing a modified roller compactor comprising an air blowing insert and a roller rim stripper, build-up of ribbons and granules in the granulator and build-up of powder and ribbons on the roller rims does not reach a level that result in a negative impact on the technical performance of the process, process yield, and product quality.

In some embodiments the modified roller compactor comprises an air blowing insert. Alternatively, in some embodiments the modified roller compactor comprises roller rim strippers. Alternatively, in some embodiments the modified roller compactor comprises an air blowing insert and roller rim strippers positioned at a lower portion of the roller rims.

The air blowing unit 270 and the roller rim strippers 250 are designed so that they do not interfere with the normal assembly of the roller compactors and do not require modifications of the standard Gerteis roller compactor parts. Furthermore, the positions of the air blowing unit and the roller rim stripper in the assembled roller compactors do not compromise the intended function of the roller compactors.

The functions of the air blowing unit 270 and the roller rim stripper are, without compromising other functionalities of the roller compactor, adapted to prevent build-up of ribbons and granules under the granulator and of powder and ribbons on the roller rims, respectively. Design variations of the air blowing unit, agitating and inducing the granule flow, and of the roller rim strippers clearing the rims mechanically are in scope of the present disclosure.

Figure 3A shows a roller rim stripper 350 which is designed for another type of roller compactor, namely the MACRO-PACTOR from Gerteis. The roller rim stripper 350 is very similar to the roller rim stripper 250 and varies from the roller rim stripper 250 in the angular position of the roller rim edge 355. Compared to the embodiment of figure 2A, the edge 355 is closer to the lowest point of the roller rim, when mounted in the roller compactor.

Figure 3B show an air blowing insert 370, which can be inserted in a roller compactor with a deeper flow channel (larger transverse dimension), i.e., the MACRO-PACTOR. Due to the larger transverse dimension of the flow channel the air blowing unit also requires a larger transverse dimension in order to effectively agitate and induce a flow of the granules below the sieve 318 and along the entire depth of the flow channel. The depth of the sieve insert is adapted to the depth of the flow channel. The sieve insert 318 is illustrated in figure 3C. In the illustrated embodiment in figure 2B, the increased transverse dimension is achieved by having two parallel tubes 371 with a number of nozzles directed to agitate the granules. The air blowing insert further comprises positioning parts (not shown on figure) adapted to ensure correct positioning of the air blowing insert 370 relative to the roller compactor (MACRO-PACTOR), the flow channel and the sieve 318. The positioning parts can be embodied as feet adapted to support the air blowing insert 370 and to be supported by the inner wall of the flow channel.

### Roller compaction of powder comprising sodium N-(8-(2-hydroxybenzoyl)amino)caprylic acid (SNAC)

During roller compaction of powder comprising SNAC, formed granules build up on the exit slides 215a.2, i.e., the wall structure portion 215a underneath the granulator. As granules continue to build up during the process, the flow towards the exit chute 203 eventually becomes blocked, which is seen in figure 2J right panel, wherein product comprising granules 50 blocks the flow. As a result hereof ribbons and granules start to build up in the granulator and later on ribbons and granules build up on the intermediate slides 215a.1 beneath the rollers, which is seen in figure 2J middle panel. The problem of accumulation propagates to the roller rims, wherein accumulated powder and ribbons on the roller rims 213, reduces the rotational speed of the roller 212a and eventually prevents the rotation of the roller. Figure 2L left panel illustrates product 50 adhered to the roller rims, the shown amount of product is not enough to slow the rotation, and in the illustrated example roller compaction has been conducted with the use of roller rim strippers ensuring that excess product on the rims is stripped off. If product builds up on the roller rims 213 it will initially fill out a clearance in the interface between the side wall 243 and the roller rims 213. If product continues to build up on the roller rim 213, the product will exert an increasing normal force against the first lower surface 243.1 (meaning 243a.1 and 243b.1) of the sidewall 243, and thereby an increasing friction force for a given rotational speed of the roller. To be more specific, the normal force between the roller rim and the side wall will increase as a function of the thickness of the product layer on the roller rim. As the normal force increases, the friction between the stationary side wall and the rotating roller will also increase. The normal force will increase, as the sidewall is flexibly mounted to the top plate 242 and biased towards the roller rims 213. Therefore, the movement of the side wall is restricted. In this situation the roller rim with the accumulated product layer functions as a break. There are at least two types of product build-up which creates problems for continuous production: the first type, (i) build-up and blockage of exit chute and, the second type, (ii) build-up on roller rims. The types of build-up have quantitative and qualitative implications for the process, and may even prevent the technical performance of the roller compaction process. Both types of product build-up reduce the yield of the process, and the second type compromise the quality of the granules. This issue has proven to be a tremendous scale-up challenge for the roller compaction process for producing SNAC granules.

### Impact on technical performance

In order to ensure the technical performance of a roller compactor, and as disclosed in the present disclosure to ensure the technical performance for the Gerteis roller compactors, for the SNAC granulation process. The roller compactor has to be partially disassembled and reassembled when product build-up occurs. Therefore, to enable large scale production of granules, the roller compactor has to be disassembled and reassembled several times. This is necessary to remove product build-up of powder, ribbons, and granules around and in the granulator and on the roller rims in order to prevent blockage of the material flow in the roller compactor and to prevent slowing down and eventually stop the rotation of the rollers. During roller compaction of the SNAC powder mixture, the roller compactor potentially has to go through a cycle of disassembling, removal of granulator build-up, and reassembling after 6^{th} minutes of manufacturing. Furthermore, every 30^{th} minute of manufacturing (i.e. 5 x 6 minutes of manufacturing) the dis- and reassembling cycle has to additionally include a cleaning of the roller rims to remove build-up on the roller rims. Each of such cleaning cycles are time consuming, as a cleaning cycle of the roller rims takes approximately 30 minutes and a cleaning cycle including the granulator takes approximately 6 hours. Each "granulator cycle", which cycle comprises 6 minutes of manufacturing and "removal of build-up below the granulator " takes approximately 6+30 minutes, and each "rim cycle", which cycle comprises 5 "granulator cycles" and "removal of build-up below granulator and cleaning of roller rims" takes approximately 180+330 minutes. Hence, large scale production of granules requiring more than 6 minutes of granulation can be shortened substantially. A "rim cycle" of 510 minutes as referred to above can instead be performed as a continuous production taking just 30 minutes resulting in a 17 fold increase in production capacity.

### Impact on yield

Build-up of ribbons and granules in the granulator and beneath the rollers potentially has to be removed every 6^{th} minutes of manufacturing. Likewise, build-up of powders and ribbons on the roller rims potentially has to be removed every 30^{th} minutes of manufacturing. These multiple build up removals of powder, ribbons, and granules to enable manufacturing of SNAC granules will significantly reduce the yield of the process because the removed powders, ribbons, and granules are scrapped.

### Impact on quality

The quality of the granules may be compromised when ribbons and granules build up in the granulator and beneath the rollers. The reason for this is that ribbons and granules might reside within the granulator for a longer time than intended which in turn results in excessive comminution of the granules and ribbons. This results in granules with a smaller particle size than intended. Furthermore, the quality of the granules may be compromised when powder and ribbons build up on the roller rims as the rotational speed of the rollers might slow down which in turn impacts the granule size, densities, and tableting properties.

### Roller compaction of SNAC granules comprising cleaning cycles

Thus in an embodiment of the present disclosure is provided a method of producing granules comprising SNAC according to the present disclosure, the method comprises: (i) providing a powder comprising SNAC, (ii) dry granulating the powder by using a roller compactor, comprising a pair of pressing rollers 212 with roller rims 213, a granulator 216, a sieve 218, and an exit slide 215a.2 below the sieve 218, wherein the dry granulation is conducted in a plurality of production cycles (iii) performing a first cleaning cycle of disassembling, cleaning and reassembling the roller compactor to clean and remove accumulated product from the exit slide 215a.2 below the granulator, (iv) performing a second cleaning cycle of disassembling, cleaning and reassembling the roller compactor to clean and remove accumulated product from the exit slide 215a.2 and slides 215a.1 below the pressing rollers 212, wherein one of the cleaning cycles are conducted after each production cycle, and wherein the first cleaning cycle is conducted at a higher frequency than the second cleaning cycle.

In an embodiment of the present disclosure, the powder, granules and intermediate products are characterized by poor flowability due to cohesive forces, or electrostatic charging. The poor flowability can be observed as a tendency to build-up below the granulator and the sieve insert and below the pair of rollers 212. The poor flowability can be measured as a production stop due to a product build-up below the granulator before the completion of a production cycle without cleaning below the granulator of a period longer than 1 minute to 120 minutes, or preferably longer than 6 minutes.

Thus in another embodiment of the present disclosure is provided a method of producing granules comprising SNAC according to the present disclosure, the method comprises: (i) providing a powder comprising SNAC, (ii) dry granulating the powder by using a roller compactor comprising a housing 210, a pair of pressing rollers 212, wherein a first pressing roller 212a comprises roller rims 213, and wherein the first pressing roller is rotatingly mounted in the housing, a granulator 216, a sieve 218, and an exit slide below the sieve 218.

Thus in an embodiment of the present disclosure, the roller compactor further comprises a rigidly mounted channel insert unit 240 comprising sidewalls adapted to be inserted upstream and adjacent to the pair of rollers 212 and for interfacing with the first pressing roller 212a, wherein the channel insert unit 240 is adapted to provide a channel portion 248 from a feeding unit 220 to a pressing space 214 between the pair of rollers 212, each of the side walls 243 comprises a first lower curved surface 243a.1, 243b.1 adapted to interface the roller rims 213, wherein a clearance is provided between the side walls 243 and the roller rims 213, and wherein the clearance is adapted to minimize friction upon rotation of the roller rims 213 but also to restrict powder from escaping the intended flow path through the pressing space 214 during the dry granulation, and wherein, in response to product build up on the roller rims 213, friction will be generated between the sidewalls and the roller rims to cause the rotational speed to slow down.

Thus in an embodiment of the present disclosure is provided a method of producing granules comprising SNAC according to the present disclosure, the method comprises: (i) providing a powder comprising SNAC, (ii) dry granulating the powder by using a roller compactor, comprising a pair of pressing rollers 212 with roller rims 213, a granulator 216, a sieve 218, and an exit slide below the sieve 218, wherein the dry granulation is conducted in a plurality of production cycles, wherein the method further comprises (iii) performing a first cleaning cycle of disassembling, cleaning and reassembling the roller compactor to clean and remove accumulated product from the exit slide below the granulator 216, (iv) performing a second cleaning cycle of disassembling, cleaning and reassembling the roller compactor to clean and remove accumulated product from the exit slide and the roller rims 213, wherein one of the cleaning cycles are conducted after each production cycle within the plurality of production cycles, and wherein the first cleaning cycle is conducted at a higher frequency than the second cleaning cycle.

In an embodiment of the present disclosure, the powder, granules and intermediate products are characterized by poor flowability due to cohesive forces, or electrostatic charging. The poor flowability can be observed as a tendency to build-up below the granulator and the sieve insert, below the pair of rollers 212, and/or on the roller rims 213. The poor flowability can be measured as a production stop due to a product build-up below the granulator before the completion of a production cycle without cleaning below the granulator of a period longer than 1 minute to 120 minutes, or preferably longer than 6 minutes, and/or as a production stop due to a product build-up on the roller rims 213 before the completion of a production cycle without cleaning of the roller rims of a period longer than 1 minute to 120 minutes, or preferably longer than 30 minutes.

### Continuous roller compaction of SNAC granules

In order to be able to produce granules comprising SNAC in an efficient manner the present disclosure provides a roller compactor adapted for extending the continuous run time of the roller compactor and/or reducing the number of cleaning cycles, when compared to a production under similar conditions using an unmodified or conventional roller compactor. By continuous production is hereby meant continuous in the sense that the production is not stopped due to the necessity of cleaning the roller compactor.

In an embodiment of the present disclosure the problem of product build-up or accumulation in the exit slide below the sieve insert and on the slides below the pressing rollers 212, is addressed, by providing an agitator for the roller compactor 200, wherein the agitator is adapted for agitating product tending to accumulate or build-up below the sieve insert 218. The agitator can be in the form of an air blowing insert 270, as described later, a vibrating plate agitating the granules and utilizing vibrational energy and gravity to lead the agitated granules toward the outlet 202, mechanical brushes or scrapers agitating the product on the slides 215a.2 by brushing or scraping. As accumulation on the slides below the sieve and the granulator is prevented, so is the upstream accumulation on the slides 215a.1 below the pressing rollers 212.

For an embodiment of the present disclosure, is provided a roller compactor 200 for production of granules by continuous dry granulation of powders, wherein the roller compactor comprises: a flow channel for conveying a product from an inlet 201 to an outlet 202 through a pressing space 214, wherein the product can be in the form of powder, ribbons or granules. The roller compactor 200 further comprises: a housing 210, a pair of rollers 212 for forming the pressing space 214 enclosed by the housing 210, wherein the pair of pressing rollers 212 comprises two pressing rollers 212a, 212b, and wherein the first pressing roller 212a comprises roller rims 213. The roller compactor 200 further comprises: a granulator 216 arranged in the housing 210 downstream to the pair of pressing rollers 212, and a sieve insert 218 mounted in the housing 210 adjacent and downstream to the granulator 216. The roller compactor 200 further comprises an agitator adapted for agitating product tending to accumulate or build-up at the exit slides 215.2 below the sieve insert 218, and thereby lead the agitated product toward the outlet 202.

Additionally, in an embodiment of the present disclosure the problem of product build-up is addressed by also providing a roller rim stripper 250 for the roller compactor 200, wherein the roller rim stripper 250 is adapted for stripping off product tending to accumulate or build-up on the roller rim 213.

In an embodiment of the present disclosure is provided a method of producing SNAC granules according to the present disclosure, the method comprises: (i) providing a powder comprising SNAC, (ii) dry granulating the powder by using a roller compactor, comprising a pair of pressing rollers 212 with roller rims 213, a granulator 216 and a sieve 218, and an outlet below the sieve 218, wherein the roller compactor further comprises an agitator adapted for agitating product tending to accumulate or build-up below the sieve insert 218 (iii) continuously during dry granulation, agitating and guiding product towards the outlet.

In an embodiment of the present disclosure, the powder, granules and intermediate products are characterized by poor flowability due to cohesive forces, or electrostatic charging. The poor flowability can be observed as a tendency to build-up below the granulator and the sieve insert and below the pair of rollers 212. The poor flowability can be measured as production stop, in a reference roller compactor without the agitator, due to a product build-up below the granulator and sieve insert before the completion of a production cycle without cleaning below the granulator of a period longer than a time within the interval 1 minute to 120 minutes, or preferably longer than 6 minutes.

For the purpose of comparison, the reference roller compactor is defined as a roller compactor wherein all the mechanical parts, with the exception of the agitator, are identical to the embodiment according to the present disclosure. In other words, the embodiment according to the disclosure is the reference roller compactor with the agitator inserted.

Alternatively, in an embodiment of the present disclosure is provided a method of producing SNAC granules according to the present disclosure, the method comprises: (i) providing a powder comprising SNAC, (ii) dry granulating the powder by using a roller compactor, comprising a pair of pressing rollers 212 with roller rims 213, a granulator 216, a sieve 218, an exit slide below the sieve 218 and an agitator adapted to agitate granules at the exit slide.

In an alternative embodiment of the present disclosure the problem of product build-up or accumulation on the roller rims, is addressed, by providing a roller rim stripper for the roller compactor 200, wherein the roller rim stripper is adapted for stripping off product tending to accumulate or build-up on the roller rims and thereby avoid the slowing down of the roller rims and the pressing rollers during production.

For an embodiment of the present disclosure, the roller compactor comprises a flow channel for conveying a product from an inlet 201 to an outlet 202 through a pressing space 214, the roller compactor further comprises a channel insert unit 240 comprising side walls 243 adapted to be inserted upstream and adjacent to the pair of rollers 212 and for interfacing with the first pressing roller 212a, wherein the channel insert unit 240 is adapted to provide a channel portion 248 from an inlet to the pressing space 214 between the pair of rollers 212, each of the side walls comprises a first lower curved surface 243a.1, 243b.1 adapted to interface with the roller rims 213, wherein a clearance is provided between the side walls and the roller rims 213, and wherein the clearance is adapted to minimize friction upon rotation of the roller rims 213 but also to restrict powder from escaping the intended flow path through the pressing space 214 during the dry granulation, and wherein, in response to product build up on the roller rims 213 friction will be generated and cause the rotational speed to slow down, wherein the roller compactor further comprises a roller rim stripper adapted for stripping off product tending to accumulate or build-up on the roller rims and thereby avoid the slowing down of the roller rims and the pressing rollers during production.

In some embodiments, the side walls are flexibly arranged to be movable in a direction away from the pressing roller. In such an embodiment the side walls are adapted to be biased towards the position adjacent to the pressing roller, and thereby provide a biasing force. In this position the small clearance between side wall and roller rims is provided. In response to the side wall being forced away from the pressing roller the biasing force will counteract.

In some embodiments, the roller rim stripper is formed as a scraper adapted for lifting adhered product off the roller rim. In some embodiments a scraper comprises an inclined surface adapted for lifting off adhered product.

In some embodiments, the roller rim stripper is formed as a blocking element adapted for blocking adhered product off the roller rim. In some embodiments a blocking element comprises a blocking surface with a blocking edge, wherein the blocking surface defines a plane normal to a tangent of the roller rim, at the position closest to the blocking edge. Before production a clearance is provided between the blocking edge and the roller rim.

In some embodiment, the roller rim strippers are positioned relative to the roller rims to strip off product from a lower portion of the roller rims, wherein the relative positioning at the lower portion of the roller rims ensure that stripped off product, will be forced away from the roller rims by gravity. Hereby, the direction of gravity defines the lower portion relative to the upper portion. The lower portion defines the 50% by mass that are positioned lower to the upper portion in the direction of gravity.

Alternatively, in an embodiment of the present disclosure is provided a method of producing SNAC granules according to the present disclosure, the method comprises: (i) providing a powder comprising SNAC, (ii) dry granulating the powder by using a roller compactor comprising a flow channel for conveying a product from an inlet 201 to an outlet 202 through a pressing space 214, a channel insert unit 240 comprising side walls 243 adapted to be inserted upstream and adjacent to the pair of rollers 212 and for interfacing with the first pressing roller 212a, wherein the channel insert unit 240 is adapted to provide a channel portion 248 from an inlet to the pressing space 214 between the pair of rollers 212, each of the side walls comprises a first lower curved surface 243a.1, 243b.1 adapted to interface with the roller rims 213, wherein a clearance is provided between the side walls and the roller rims 213, and wherein the clearance is adapted to minimize friction upon rotation of the roller rims 213 but also to restrict powder from escaping the intended flow path through the pressing space 214 during the dry granulation, and wherein, in response to product build up on the roller rims 213 friction will be generated and cause the rotational speed to slow down, wherein the roller compactor further comprises a roller rim stripper adapted for stripping off product tending to accumulate or build-up on the roller rims and thereby avoid the slowing down of the roller rims and the pressing rollers during production, wherein the method further comprises (iii) continuously during dry granulation, stripping accumulated product of the roller rims 213.

In an embodiment, the powder, granules and intermediate products are characterized by poor flowability due to cohesive forces, or electrostatic charging. The poor flowability can be observed as a tendency to build-up below the granulator and the sieve insert, below the roller rims 213. The poor flowability can be measured as a production stop due to a production stop due to a product build-up on the roller rims 213, in a reference roller compactor without roller rim strippers, before the completion of a production cycle without cleaning of the roller rims of a period longer than 1 minute to 120 minutes, or preferably longer than 30 minutes.

For the purpose of comparison, the reference roller compactor is defined as a roller compactor wherein all the mechanical parts, with the exception of the roller rim strippers, are identical to the embodiment according to the present disclosure. In other words, the embodiment according to the disclosure is the reference roller compactor with the roller rim stripper inserted.

In an alternative embodiment of the present disclosure the problem of product build-up or accumulation in the exit slide below the sieve insert, on the slides below the pressing rollers 212 and accumulation on the roller rims, is addressed, by providing an agitator 270 for the roller compactor 200 and a roller rim stripper 250, wherein the agitator is adapted for agitating product tending to accumulate or build-up on the exit slide below the sieve insert 218. As accumulation on the slides below the sieve and the granulator is prevented, so is the upstream accumulation on the slides 215a.1 below the pressing rollers 212. The roller rim stripper is adapted for stripping off product tending to accumulate or build-up on the roller rims and thereby avoid the slowing down of the roller rims and the pressing rollers during production. Using an agitator will minimize the tendency of product to accumulate on the roller rims, but over long production cycles a significant amount may accumulate and slow down the roller rims and thereby the pressing rollers.

In some embodiments, the agitator can be in the form of an air blowing insert 270, as described later, a vibrating plate agitating the granules and utilizing vibrational energy and gravity to lead the agitated granules toward the outlet 202, mechanical brushes or scrapers agitating the product on the slides 215a.2 by brushing or scraping.

In an embodiment of the present disclosure is provided a method of producing SNAC granules according to the present disclosure, the method comprises: (i) providing a powder comprising SNAC, (ii) dry granulating the powder by using a roller compactor, comprising a pair of pressing rollers 212 with roller rims 213, a granulator 216, a sieve 218, and exit slides below the sieve 218, wherein the roller compactor further comprises an agitator adapted for agitating product tending to accumulate or build-up below the sieve insert 218, and a roller rim stripper adapted for stripping off product tending to accumulate or build-up on the roller rim 213 (iii) continuously during dry granulation, agitating and guiding product on the exit slides towards an outlet, and stripping off accumulated product from the roller rim 213.

In some embodiment of the present disclosure, the powder, granules and intermediate products are characterized by poor flowability due to cohesive forces, or electrostatic charging. The poor flowability can be observed as a tendency to build-up below the granulator and the sieve insert, below the pair of rollers 212, and/or on the roller rims 213. The poor flowability can be measured as a production stop due to a product build-up below the granulator, in a reference roller compactor without an agitator and the roller rim strippers, before the completion of a production cycle without cleaning below the granulator of a period longer than 1 minute to 120 minutes, or preferably longer than 6 minutes, and/or as a production stop due to a product build-up on the roller rims 213, in a reference roller compactor without the agitator and the roller rim strippers, before the completion of a production cycle without cleaning of the roller rims of a period longer than 1 minute to 120 minutes, or preferably longer than 30 minutes.

For the purpose of comparison, the reference roller compactor is defined as a roller compactor wherein all the mechanical parts, with the exception of the agitator and the roller rim strippers, are identical to the embodiment according to the present disclosure. In other words, the embodiment according to the disclosure is the reference roller compactor with the agitator and the roller rim stripper inserted, as illustrated in figure 2A.

In some embodiments according to the present disclosure, the slides 215a.2 below the sieve comprises an inclined surface with respect to the direction of gravity, and in some embodiments the problem of accumulation on the slides can be addressed by increasing the inclination.

In some embodiments according to the present disclosure, the slides215a.1 below the pressing rollers comprises an inclined surface with respect to the direction of gravity, and in some embodiments the problem of accumulation on the slides can be addressed by increasing the inclination.

## Claims

1. A method of producing ribbons or granules comprising a salt of N-(8-(2-hydroxybenzoyl)amino)caprylic acid, wherein the method comprises:
a) providing a powder comprising a salt of N-(8-(2-hydroxybenzoyl)amino)caprylic acid;
b) compacting the powder into ribbons by using a roller compactor wherein the roller compactor comprises: an inlet (201) and an outlet (202), a pair of pressing rollers (212) comprising a first (212a) and a second pressing roller (212b), wherein the first pressing roller (212a) comprises roller rims (213) and wherein the roller compactor further comprises roller rim strippers (250).

2. The method according to claim 1, wherein the roller compactor further comprises a granulator (216), a sieve (218), and exit slides (215.2) below the sieve (218) leading towards the outlet (202).

3. The method according to claim 1 or 2, wherein the roller rim strippers (250) are adapted for stripping off material tending to accumulate or build-up on the roller rims (213), and thereby lead the stripped off material towards the outlet (202).

4. The method according to any of the previous claims, wherein the roller compactor further comprises a channel insert unit (240) adapted to interface the roller rims (213) and provide a clearance between the channel insert unit and the roller rims (213).

5. The method according to any of the previous claims, wherein the roller compactor further comprises a roller scraper (230) with a roller scraper edge (232).

6. The method according to any of the previous claims, wherein the roller rim stripper (250) comprises a stripping edge (255) and wherein said stripping edge is less than 180 degrees apart from the pressing space on the roller rim circumference in the direction of rotation.

7. The method according to claim 5 and 6, wherein the roller scraper edge (232) and the stripping edge (255) are within 90 degrees on the circumference of the pressing roller/roller rim.

8. The method according to any of the previous claims 5-7, wherein the roller scraper edge (232) and stripping edge (255) are both less than 90 degrees away from the pressing space on the circumference of the pressing roller/roller rim in the direction of rotation.

9. The method according to any of the previous claims, wherein the roller rim strippers (250) extend over more than 45 degrees on the circumferential of the roller rims 213.

10. The method according to any of the previous claims 4-9 , wherein the roller compactor comprises: a flow channel for conveying a material from an inlet (201) to an outlet (202) through a pressing space (214) formed by the pair or of rollers (212), wherein the roller compactor (200) further comprises: a housing (210) enclosing the channel insert unit, the pressing space and optionally the granulator.

11. The method according to any of the previous claims 2-10, wherein the roller compactor further comprises an agitator (270) adapted for agitating material tending to accumulate below the sieve (218).

12. The method according to any of the previous claims, wherein the method further comprises continuously during the compaction, stripping off material accumulating on the roller rims (213) and leading the stripped material towards the outlet 202.

13. The method according to any of the previous claims, wherein the ribbons or granules comprises sodium N-(8-(2-hydroxybenzoyl)amino)caprylate and a lubricant, such as magnesium stearate.

14. A method of producing a solid pharmaceutical composition comprising
a) producing granules comprising a salt of N-(8-(2-hydroxybenzoyl)amino)caprylic acid by using a method according to any of the claims 1-13
b) preparing said solid pharmaceutical formulation using the granules obtained by a).

15. The method according to claim 14, wherein the solid pharmaceutical composition comprises a GLP-1 receptor agonist such as semaglutide.

## Patentansprüche

1. Verfahren zur Herstellung von Bändern oder Granulaten, umfassend ein Salz von N-(8-(2-hydroxybenzoyl)amino)caprylsäure, wobei das Verfahren umfasst:
a) Bereitstellen eines Pulvers, das ein Salz von N-(8-(2-hydroxybenzoyl)amino)caprylsäure umfasst;
b) Verdichten des Pulvers zu Bändern unter Verwendung eines Walzenkompaktors, wobei der Walzenkompaktor umfasst: einen Einlass (201) und einen Auslass (202), ein Paar Presswalzen (212), das eine erste (212a) und eine zweite Presswalze (212b) umfasst, wobei die erste Presswalze (212a) Walzenränder (213) umfasst und wobei der Walzenkompaktor ferner Walzenrandabstreifer (250) umfasst.

2. Verfahren nach Anspruch 1, wobei der Walzenkompaktor ferner einen Granulator (216), ein Sieb (218) und Schüttrinnen (215.2) unterhalb des Siebes (218) umfasst, die zu dem Auslass (202) führen.

3. Verfahren nach Anspruch 1 oder 2, bei dem die Walzenrandabstreifer (250) zum Abstreifen von Material geeignet sind, das dazu neigt, sich an den Walzenrändern (213) anzusammeln oder aufzubauen, und dadurch das abgestreifte Material in Richtung des Auslasses (202) führen.

4. Verfahren nach einem der vorhergehenden Ansprüche, bei dem der Walzenkompaktor ferner eine Kanalzufuhreinheit (240) umfasst, die dazu ausgelegt ist, eine Schnittstelle zu den Walzenrändern (213) zu bilden und einen Abstand zwischen der Kanalzufuhreinheit und den Walzenrändern (213) bereitzustellen.

5. Verfahren nach einem der vorhergehenden Ansprüche, wobei der Walzenkompaktor ferner einen Walzenabstreifer (230) mit einer Walzenabstreiferkante (232) umfasst.

6. Verfahren nach einem der vorhergehenden Ansprüche, wobei der Walzenrandabstreifer (250) eine Abstreifkante (255) aufweist und wobei diese Abstreifkante in der Drehrichtung weniger als 180 Grad von dem Pressraum auf dem Umfang des Walzenrandes entfernt ist.

7. Verfahren nach Anspruch 5 und 6, wobei die Walzenabstreifkante (232) und die Abstreifkante (255) innerhalb von 90 Grad auf dem Umfang der Presswalze / des Walzenrandes liegen.

8. Verfahren nach einem der vorhergehenden Ansprüche 5 bis 7, wobei die Walzenabstreiferkante (232) und die Abstreifkante (255) beide in Drehrichtung jeweils weniger als 90 Grad von dem Pressraum auf dem Umfang der Presswalze / des Walzenrandes entfernt sind.

9. Verfahren nach einem der vorhergehenden Ansprüche, bei dem sich die Walzenrandabstreifer (250) über mehr als 45 Grad auf dem Umfang der Walzenränder 213 erstrecken.

10. Verfahren nach einem der vorhergehenden Ansprüche 4 bis 9, wobei der Walzenkompaktor umfasst: einen Strömungskanal zum Fördern eines Materials von einem Einlass (201) zu einem Auslass (202) durch einen Pressraum (214), der durch das Paar von Walzen (212) gebildet wird, wobei der Walzenkompaktor (200) ferner umfasst: ein Gehäuse (210), das die Kanalzufuhreinheit, den Pressraum und optional den Granulator umschließt.

11. Verfahren nach einem der vorhergehenden Ansprüche 2 bis 10, wobei der Walzenkompaktor ferner ein Rührwerk (270) umfasst, das zum Rühren von Material ausgelegt ist, das dazu neigt, sich unter dem Sieb (218) anzusammeln.

12. Verfahren nach einem der vorhergehenden Ansprüche, wobei das Verfahren ferner umfasst, während des Verdichtens kontinuierlich Material abzustreifen, das sich an den Walzenrändern (213) ansammelt, und das abgestreifte Material in Richtung des Auslasses (202) zu führen.

13. Verfahren nach einem der vorhergehenden Ansprüche, wobei die Bänder oder Granulate Natrium-N-(8-(2-hydroxybenzoyl)amino)caprylat und ein Gleitmittel, wie z. B. Magnesiumstearat, umfassen.

14. Verfahren zur Herstellung einer pharmazeutischen Feststoffzusammensetzung, umfassend:
a) Herstellen von Granulaten, die ein Salz von N-(8-(2-hydroxybenzoyl)amino)caprylsäure umfassen, unter Verwendung eines Verfahrens nach einem der Ansprüche 1 bis 13.
b) Herstellen der pharmazeutischen Feststoffformulierung unter Verwendung der durch a) erhaltenen Granulate.

15. Verfahren nach Anspruch 14, wobei die pharmazeutische Feststoffzusammensetzung einen GLP-1-Rezeptoragonisten, wie z. B. Semaglutid, umfasst.

## Revendications

1. Procédé de production de rubans ou de granules comprenant un sel d'acide N-(8-(2-hydroxybenzoyl)amino)caprylique, dans lequel le procédé comprend :
a) la fourniture d'une poudre comprenant un sel d'acide N-(8-(2-hydroxybenzoyl)amino)caprylique ;
b) le compactage de la poudre en rubans à l'aide d'un compacteur à rouleaux, le compacteur à rouleaux comprenant : une entrée (201) et une sortie (202), une paire de rouleaux presseurs (212) comprenant un premier rouleau presseur (212a) et un deuxième rouleau presseur (212b), dans lequel le premier rouleau presseur (212a) comprend des rebords de rouleau (213), et dans lequel le compacteur à rouleaux comprend en outre des débourreurs de rebord de rouleau (250).

2. Procédé selon la revendication 1, dans lequel le compacteur à rouleaux comprend en outre un granulateur (216), un tamis (218) et des glissières de sortie (215.2) au-dessous du tamis (218) conduisant vers la sortie (202).

3. Procédé selon la revendication 1 ou 2, dans lequel les débourreurs de rebord de rouleau (250) sont adaptés pour débourrer la matière tendant à s'accumuler ou à s'agglomérer sur les rebords de rouleau (213), et ainsi conduire la matière débourrée vers la sortie (202).

4. Procédé selon l'une quelconque des revendications précédentes, dans lequel le compacteur à rouleaux comprend en outre une unité d'insertion de canal (240) adaptée à s'interfacer avec les rebords de rouleau (213) et à fournir un dégagement entre l'unité d'insertion de canal et les rebords de rouleau (213).

5. Procédé selon l'une quelconque des revendications précédentes, dans lequel le compacteur à rouleaux comprend en outre un racloir à rouleau (230) avec une arête de racloir à rouleau (232).

6. Procédé selon l'une quelconque des revendications précédentes, dans lequel le débourreur de rebord de rouleau (250) comprend une arête de débourrage (255) et dans lequel ladite arête de débourrage est séparée de moins de 180 degrés de l'espace de pressage sur la circonférence du rebord de rouleau dans la direction de rotation.

7. Procédé selon les revendications 5 et 6, dans lequel l'arête de racloir à rouleau (232) et l'arête de débourrage (255) sont à moins de 90 degrés sur la circonférence du rouleau presseur/rebord de rouleau.

8. Procédé selon l'une quelconque des revendications précédentes 5 à 7, dans lequel l'arête de racloir à rouleau (232) et l'arête de débourrage (255) sont toutes deux éloignées de moins de 90 degrés de l'espace de pressage sur la circonférence du rouleau presseur/rebord de rouleau dans la direction de rotation.

9. Procédé selon l'une quelconque des revendications précédentes, dans lequel les débourreurs de rebord de rouleau (250) s'étendent sur plus de 45 degrés sur la circonférence des rebords de rouleau 213.

10. Procédé selon l'une quelconque des revendications précédentes 4 à 9, dans lequel le compacteur à rouleaux comprend : un canal d'écoulement destiné à acheminer une matière depuis une entrée (201) vers une sortie (202) à travers un espace de pressage (214) formé par la paire de rouleaux (212), dans lequel le compacteur à rouleaux (200) comprend en outre : un boîtier (210) enfermant l'unité d'insertion de canal, l'espace de pressage et facultativement le granulateur.

11. Procédé selon l'une quelconque des revendications précédentes 2 à 10, dans lequel le compacteur à rouleaux comprend en outre un agitateur (270) adapté pour agiter la matière tendant à s'accumuler au-dessous du tamis (218).

12. Procédé selon l'une quelconque des revendications précédentes, dans lequel le procédé comprend en outre, de manière continue pendant le compactage, le débourrage de la matière s'accumulant sur les rebords de rouleau (213) et le fait de conduire la matière débourrée vers la sortie (202).

13. Procédé selon l'une quelconque des revendications précédentes, dans lequel les rubans ou granules comprennent du N-(8-(2-hydroxybenzoyl)amino)caprylate de sodium et un lubrifiant, tel que le stéarate de magnésium.

14. Procédé de production d'une composition pharmaceutique solide comprenant
a) la production de granules comprenant un sel d'acide N-(8-(2-hydroxybenzoyl)amino)caprylique en utilisant un procédé selon l'une quelconque des revendications 1 à 13
b) la préparation de ladite formulation pharmaceutique solide en utilisant les granules obtenus en a).

15. Procédé selon la revendication 14, dans lequel la composition pharmaceutique solide comprend un agoniste du récepteur GLP-1, tel que le sémaglutide.
